# EUROPEAN PATENT SPECIFICATION

(11) **EP 1 036 783 B1**
(45) Date of publication and mention of the grant of the patent: **21.05.2003**
(21) Application number: 00301864.5
(22) Date of filing: 07.03.2000
(51) Int. Cl.: C07C 69/30, C07C 67/28, A61K 31/22, A61P 3/06

(54) **Crystalline hydrated dihydroxy open-acid simvastatin calcium salt**
Wasserhaltiges, kristallines Calcium-Salz der Simvastatin Dihydroxy offene Säure
Sel cristallin hydraté de calcium de la dihydroxy-simvastatine sous sa forme acide ouverte

(30) Priority: 08.03.1999 US 123247 P; 09.03.1999 US 264745; 02.02.2000 US 2627 P
(43) Date of publication of application: 20.09.2000
(73) Proprietor: MERCK & CO. INC., Rahway New Jersey 07065-0900 (US)
(72) Inventor: Tillyer, Richard D., New Jersey 07065 (US); Reider, Paul J., Rahway, New Jersey 07065 (US); Grabowski, Edward J. J., Rahway, New Jersey 07065 (US); Xu, Feng, Rahway, New Jersey 07065 (US)
(74) Representative: Thompson, John Dr.

(56) References cited:
- US-A- 4 444 784
- US-A- 5 223 415
- US-A- 5 273 995

## Description

The instant invention relates to a particular salt form of simvastatin, which is an inhibitor of 3-hydroxy-3-methylglutaryl coenzyme A (HMG-CoA) reductase. More particularly, the invention relates to a particular crystalline hydrated form of the calcium salt of dihydroxy open acid simvastatin referred to herein as compound I.

It has been clear for several decades that elevated blood cholesterol is a major risk factor for coronary heart disease (CHD), and many studies have shown that the risk of CHD events can be reduced by lipid-lowering therapy. Simvastatin (ZOCOR®; see US Patent No. 4,444,784) is a commercially successful example of the variety of known semi-synthetic and totally synthetic plasma cholesterol lowering agents referred to as HMG-CoA reductase inhibitors. The structural formulas of known HMG-CoA reductase inhibitors are described at page 87 of M. Yalpani, "Cholesterol Lowering Drugs", Chemistry & Industry, pp. 85-89 (5 February 1996). The HMG-CoA reductase inhibitors typified by simvastatin belong to a structural class of compounds which contain a moiety which can exist as either a 3-hydroxy lactone ring or as the corresponding ring opened dihydroxy open-acid, and are often referred to as "statins". An illustration of the lactone portion of a statin and its corresponding open-acid form is shown below. 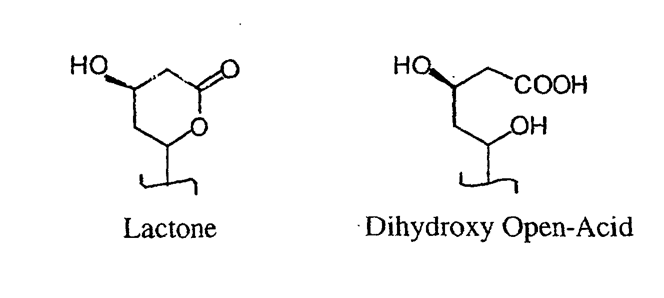 Salts of the dihydroxy open-acid can be prepared, and in fact several of the marketed statins are administered as the dihydroxy open acid salt forms. Simvastatin is marketed worldwide in its lactonized form. Simvastatin is shown as structural formula III, below.

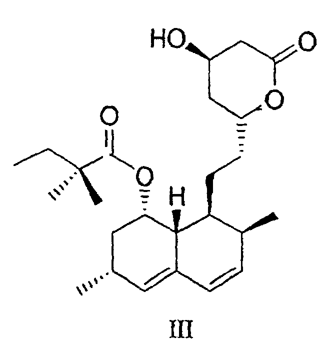

The lactonized forms of the statins are not active inhibitors of HMG-CoA reductase, but the dihydroxy open acid forms are. It is known that condensation of the dihydroxy open acid form of statins to the corresponding lactonized form occurs under acidic conditions, that is at about pH 4 or under. Therefore, due to the low gastric pH of the stomach, a statin conventionally administered by oral dosing in its lactone form will remain largely in its lactone form in the stomach. The vast majority of the drug will still be in the lactone form at the time of absorption from the intestine following oral dosing with the lactone. After absorption, the lactone enters the liver and it is in the hepatocytes that the lactone can be metabolized to the active open acid form, a reaction catalyzed by two hepatic esterases or "lactonases," one which is in the cytosolic and the other in the microsomal fraction. Once in the blood there is an additional plasma esterase which can also hydrolyze the lactone to the open acid. There may be some minimal chemical, i.e., non-enzymatic, hydrolysis that occurs in blood or in the liver; however, at the pH in blood and liver, there should not be any lactonization, i.e., conversion of open acid back to the lactone.

A statin conventionally administered by oral dosing in its dihydroxy open acid form or a pharmaceutically acceptable salt or ester thereof will tend to convert to its lactone form in the acidic environment of the stomach, so that a mixture of the open ring and the corresponding closed ring forms will co-exist there. For example, see M.J. Kaufman, International Journal of Pharmaceutics, 1990, 66 (Dec 1), p. 97-106, which provides hydrolysis data that are used to simulate the extent of drug degradation that occurs in acidic gastric fluids following oral administration of several structurally related hypocholesterolemic agents, including simvastatin. Therefore, even after conventional oral dosing with a dihydroxy open acid statin or a salt or ester thereof, a mixture of the open acid and the corresponding lactone form of the drug could exist by the time of absorption from the intestine.

Several procedures have been published describing ways to make the semi-synthetic analog simvastatin from the naturally occurring compound lovastatin, and most proceed through a lactone ring opening step at some point in the process and sometimes formation of a salt at the resulting carboxy acid, and end with a ring-closing step in order to make the final simvastatin product. Typical procedures for preparing simvastatin from lovastatin are described in US Patents 4,820,850, 4,444,784 and 4,582,915.

Some salt forms of dihydroxy open acid simvastatin have been previously described, for example the ammonium salt and the potassium salt which were used as intermediates in the synthetic processes referred to above. Chemical Abstracts also discloses various salt forms of dihydroxy open acid simvastatin, including a calcium salt having Registry No. 151006-18-7, which was referenced from a genus disclosed in U.S Patent No. 5,223,415. However, there are no specific calcium salts exemplified in that patent, and the generic description of salt preparation taught in that patent is non-enabling for obtaining a crystalline form of the calcium salt of dihydroxy open acid simvastatin but instead would lead to an amorphous calcium salt product.

US Patent No 5,273,995 discloses the calcium salt of the dihydroxy open acid form of a diarylpyrrole-based statin.

Since becoming available, millions of doses of simvastatin have been administered and this drug has developed an excellent safety record. However, as noted in the Physician's Desk Reference (PDR), in rare instances myopathy has been associated with the use of all statins, including simvastatin. The mechanism for statin-related myopathy is currently poorly understood. It is also known that many drugs, including certain statins such as simvastatin, are metabolized in the liver and intestine by the cytochrome P450 3A4 (CYP 3A4) enzyme system. As also noted in the PDR, there are adverse drug interaction concerns if a potent inhibitor of CYP3A4, such as itraconazole, and a CYP3A4-metabolized statin are used together, and some cases of myopathy were found to have occurred in patients taking such a drug combination. Simvastatin has been administered to over 20 million patients worldwide in the past 11 years and has been demonstrated to be remarkably safe. However, the very low risk of myopathy is substantially increased when simvastatin is given together with potent inhibitors of CYP3A4. While the overall safety record for simvastatin is exceptional, it would be desirable to further optimize its safety profile by reducing the potential for drug interactions with inhibitors of CYP3A4. It would also be desirable to further reduce the already low rate of occurrence of myopathy associated with the use of simvastatin.

Applicants have now found that dihydroxy open acid simvastatin may be less reliant on CYP3A4 metabolism than its closed ring lactonized counterpart. The instant invention provides a novel crystalline hydrated form of the calcium salt of dihydroxy open acid simvastatin which has been found to be a pharmaceutically suitable salt form having desirable physical properties for formulation into an anti-hypercholesterolemic drug composition.

The instant invention accordingly provides novel HMG-CoA reductase inhibitors which are crystalline hydrated forms of the calcium salt of dihydroxy open acid simvastatin, and particularly the compound referred to herein as compound I.

This invention also relates to the use of the crystalline hydrated forms of the calcium salt of dihydroxy open acid simvastatin, particularly compound I, for inhibiting HMG-CoA reductase, as well as for treating and/or reducing the risk for diseases and conditions affected by inhibition of HMG-CoA reductase; and also provides pharmaceutical formulations, including conventional rapid-release, delayed-release and time controlled-release formulations, including the GEM drug delivery device and enterically coated dosage forms, that can be used with the compounds. Also provided by the present invention is a process for making compound I.

The invention will be more particularly understood by reference to the accompanying drawings, in which:

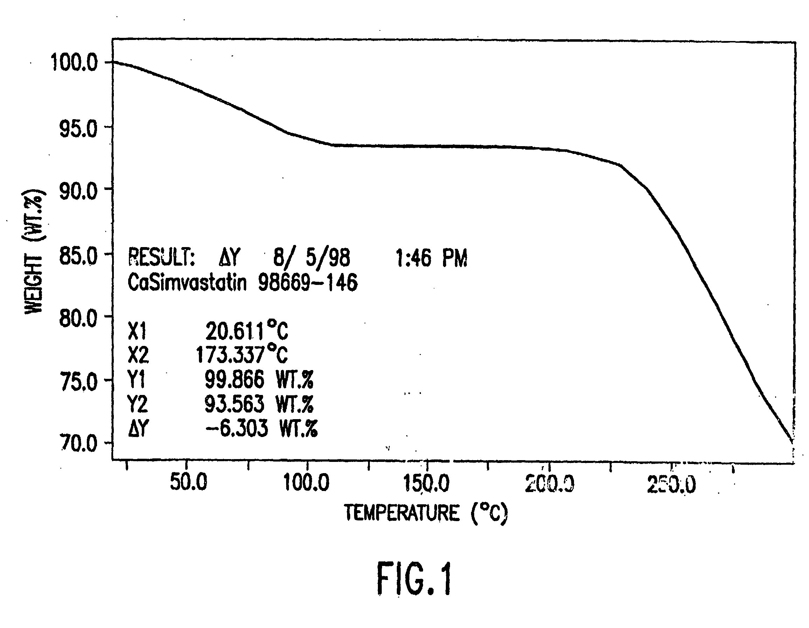

Figure 1 is a thermogravimetry (TG) weight loss curve for compound I obtained under a nitrogen flow at a heating rate of 10°C/minute.
Figure 2 is a differential scanning calorimetry (DSC) curve for compound I obtained under a nitrogen flow bubbled through 16.0°C water at a heating rate of 10°C/minute in an open cup.

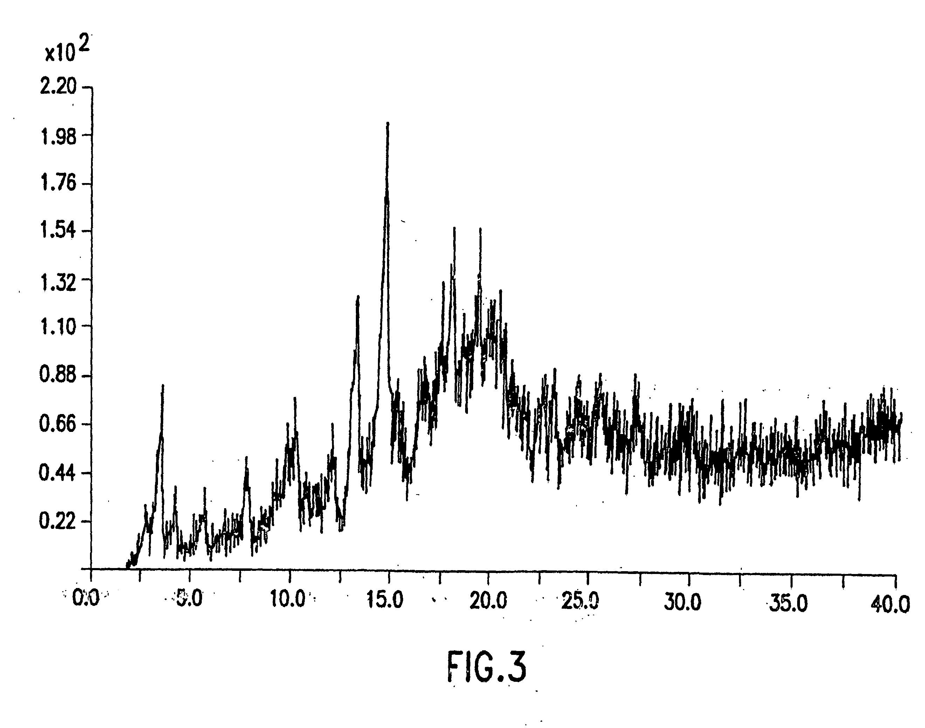

Figure 3 is an x-ray powder diffraction (XRPD) pattern for compound I. The XRPD pattern was obtained using CuKα radiation. The ordinate or Y-axis is x-ray intensity (cpm) and the abscissa or X-axis is the angle two-theta (2θ) in degrees.
Figure 4 is a solid-state ¹³C nuclear magnetic resonance spectrum for Compound I.

The term "statin(s)" as used herein is intended to be defined as inhibitors of HMG-CoA reductase which belong to a structural class of compounds that contain a moiety which can exist as either a 3-hydroxy lactone ring or as the corresponding ring opened dihydroxy open acid,
wherein the lactone portion of the statin and its corresponding dihydroxy open-acid form is shown below. 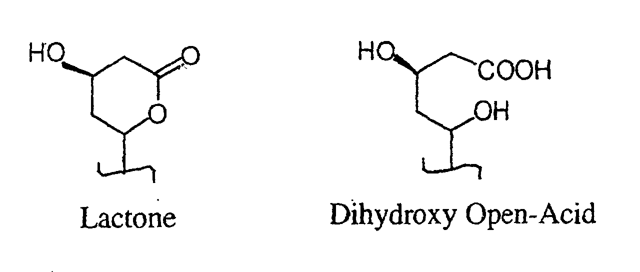 All hydrates, solvates and polymorphic crystalline forms of HMG-CoA reductase inhibitors having the above-described lactone/dihydroxy open acid moiety are included within the scope of the term "statin(s)". Pharmaceutically acceptable salts and esters of the dihydroxy open-acid statins are included within the scope of the term "statin(s)".

Statins inhibit HMG-CoA reductase in their dihydroxy open acid form. Compounds which have inhibitory activity for HMG-CoA reductase can be readily identified by using assays well-known in the art. For example, see the assays described or cited in U.S. Patent 4,231,938 at col. 6, and WO 84/02131 at pp. 30-33.

The present invention provides in one aspect the crystalline hydrated forms of the calcium salt of dihydroxy open acid simvastatin, and more particularly the hydrated calcium salt of dihydroxy open acid simvastatin referred to herein as compound I.

In another aspect, the present invention provides a pharmaceutical composition comprising a crystalline hydrated form of the calcium salt of dihydroxy open acid simvastatin, and more particularly the hydrated calcium salt thereof referred to herein as compound I, in association with a pharmaceutically acceptable carrier.

In accordance with this aspect of the invention, there are provided methods for reducing the amount of lactonized statin formed and absorbed in the body after oral administration of a dihydroxy open acid statin in order to achieve enhanced clinical benefits. A way to achieve this is to administer the dihydroxy open acid statin in a delayed-release pharmaceutical dosage form. A delayed-release pharmaceutical dosage form as defined herein
is an orally administrable pharmaceutical dosage form or device that does not release a substantial amount of the active compound, i.e., the dihydroxy open-acid statin, until after the dosage form has passed through the stomach. Therefore, substantial release of the active compound would initially occur after entry into the duodenum. By "substantial release", it is intended that 90% or more by weight of the active compound in the delayed-release dosage form is released after entry into the duodenum, and that 10% or less by weight of the active compound in the delayed-release dosage form is released in the stomach, i.e., the geometric mean ratio of the plasma AUC (area under the curve) of active vs. total HMG-CoA reductase inhibitory activity will be greater than or equal to 90%. Particularly, the amount of active compound released in the stomach before entry into the duodenum is 5% or less by weight, ie., the geometric mean ratio of the plasma AUC of active vs. total HMG-CoA reductase inhibitory activity will be greater than or equal to 95%, and more particularly the amount of active compound released in the stomach before entry into the duodenum is 1% or less by weight, ie., the geometric mean ratio of the plasma AUC of active vs. total HMG-CoA reductase inhibitory activity will be greater than or equal to 99%.

It is to be understood that metabolism of the dihydroxy open acid statin will occur, primarily in the liver, after orally dosing in a delayed-release dosage form. However, since lactonization of the dihydroxy open acid statin would have been substantially avoided by use of a delayed release dosage form, the active and inactive metabolites that are formed will also be in the dihydroxy open acid form. In essence, if the dihydroxy open acid statin is administered in a delayed release dosage form, the internal exposure to lactonized parent compound and also to lactonized active and inactive metabolites will be minimized.

One example of a suitable delayed-release dosage form is a pH-dependent enterically coated dosage form. The enteric coating will not dissolve in the acidic gastric environment, but will dissolve in the higher pH environment of the duodenum. An enterically coated dosage form will therefore not permit release of any significant amount of the active compound from the dosage form in the stomach, but once the enteric coating dissolves in the duodenum, the active compound will be released. Suitable compositions for enteric coatings that can be used with the present invention are known to those of ordinary skill in the pharmaceutical arts; for example, see L. Lachman, The Theory and Practice of Industrial Pharmacy, 3rd Ed., H. Liebermann and J. Kanig contributors (Lea & Febiger, 1986). An example of a suitable enteric coating includes but is not limited to SURETERIC® WHITE sold by Colorcon which is composed of polyvinyl acetate phthalate, titanium dioxide, talc, colloidal silicon dioxide, triethyl acetate, polyethylene glycol; sodium bicarbonate, purified stearic acid, and sodium alginate. Many other suitable enteric coating materials are commercially available and are known in the art. Additional coatings employed in the preparation of the dosage form, such as those used to provide an elegant, aesthetically pleasing final product or for other purposes, may be applied before or after, or before and after, application of the enteric coating.

Suitable enterically coated pharmaceutical dosage forms for use with this invention include enterically coated conventional rapid-release (also referred to as immediate-release) pharmaceutical dosage forms wherein the drug is relatively rapidly released once the enteric coating is breached, and enterically coated time-controlled release dosage forms such as but not limited to an enterically coated GEM delivery device, described below. Time controlled-release dosage forms are also well known in the pharmaceutical art, and are designed so as to slowly release the active compound in the body over a period of time, for example over a period of from about 6 to 24 hours. Use of an enteric coated time controlled-release dosage form may be preferred with more potent dosage amounts of a dihydroxy open acid statin so as to lower the systemic exposure to the active statin. Whether the dosage form is an enterically coated rapid-release or time-controlled release dosage form, the enteric coating will prevent release of any substantial amount of the active compound from the dosage form in the stomach.

Enterically coated pharmaceutical dosage forms also include but are not limited to those wherein the dosage form or unit is comprised of the dihydroxy open acid statin in a tablet, capsule or the like that is surrounded by an enteric overcoating, and those wherein the dosage form or unit is a tablet, capsule or the like comprised of enterically coated granules of the dihydroxy open acid statin. Where the dosage form is surrounded by an enteric overcoat, the enteric coating may be the outermost external coating on the dosage form, or there may be one or more additional finish coatings applied over the enteric coat. In a more limited embodiment, the delayed-release dosage unit contains enterically coated granules of the drug, provided in particular that the statin is not dosed in a single pharmaceutical dosage form or unit comprised of enteric coated granules of the statin and enteric coated or non-enteric coated granules of aspirin.

An example of a delayed-release dosage form that also functions as a time controlled-release dosage form is described in U.S Patent No. 5,366,738. The controlled-release drug delivery device described in U.S Patent No. 5,366,738 is known as a gel extrusion module (GEM) delivery device. The GEM device is a drug delivery device for the controlled in situ production and release of a dispersion containing a beneficial agent such as a pharmaceutical drug comprising:
(A) a compressed core prepared from an admixture comprising:
   (i) a therapeutically effective amount of the beneficial agent; and
   (ii) a polymer which upon hydration forms gelatinous microscopic particles; and
(B) a water insoluble, water impermeable polymeric coating comprising a polymer and a plasticizer, which surrounds and adheres to the core, the coating having a plurality of formed apertures exposing between about 1 and about 75% of the core surface; and wherein the release rate of the beneficial agent from the device is a function of the number and size of the apertures.

In the GEM device, the polymer inside the compressed core is preferably selected from sodium polyacrylate, carboxypolymethylenes and the pharmaceutically acceptable salts thereof such as a sodium salt, wherein the carboxypolymethylenes are prepared from acrylic acid crosslinked with allylethers of sucrose or pentaerythritol, and more preferably it is selected from carboxypolymethylenes prepared from acrylic acid crosslinked with allylethers of sucrose or pentaerythritol, and the pharmaceutically acceptable salts thereof. Most preferably, CARBOPOL® 974P and pharmaceutically acceptable salts thereof, particularly the sodium salt, is used as the polymer inside the compressed core. In addition, the compressed core may also contain one or more polymer hydration modulating agents, anti-oxidants, lubricants, fillers and excipients. An optional subcoating may be applied to the compressed core prior to application of the water insoluble coating as an aid in the manufacturing process. The subcoating may be comprised of, for example, hydroxypropyl cellulose and hydroxypropylmethylcellulose.

The water insoluble, water impermeable polymeric coating is preferably comprised of (1) a polymer selected from polyvinyl chloride, cellulose acetate, cellulose acetate butyrate, ethylcellulose and combinations of these polymers; and (2) a plasticizer selected from diethylphthalate, dibutylsebacate and triethylcitrate. More preferably, the polymeric coating is comprised of cellulose acetate butyrate and triethyl citrate. The GEM device does not function as an osmotic drug delivery device, hence the release function of the device depends on passage of fluids from the external environment of the body to the internal environment of the compressed core through the formed apertures. It is intended that the terms "water insoluble, water impermeable" used to describe the polymeric coating define a coating which is essentially water insoluble and water impermeable, meaning that the polymeric coating allows minimal to no passage of water through the coating from the external environment of the body to the internal environment of the compressed core, except for the fluid passage that occurs through the drilled apertures, during the period of time the drug is being released from the GEM device in the body. Any minimal amount of water that does pass through the water insoluble, water impermeable polymeric coating is insubstantial and does not significantly contribute to the function of the GEM device, i.e. the release rate of the drug through the apertures. Rather the release rate of the drug from the GEM device is primarily a function of the number and size of the apertures on the device.

For an elegant, aesthetically pleasing final product, an outer finish coat may finally be applied to the GEM delivery device containing colorants, waxes, and the like. The GEM device can also be enterically coated, either before or after the application of additional finish coatings. Even without enteric coating, extrusion of the polymer which carries the drug out from inside the compressed core of the GEM device does not occur to a substantial extent in the acidic pH of the stomach, therefore substantial release of the drug should not occur in the stomach. Further details and examples of the GEM delivery device are described in US Patent No. 5,366,738.

The term "patient" includes mammals, especially humans, who use the instant active agents for the prevention or treatment of a medical condition. Administering of the drug to the patient includes both self-administration and administration to the patient by another person. The patient may be in need of treatment for an existing disease or medical. condition, or may desire prophylactic treatment to prevent or reduce the risk for'diseases and medical conditions affected by inhibition of HMG-CoA reductase.

The term "therapeutically effective amount" is intended to mean that amount of a drug or pharmaceutical agent that will elicit the biological or medical response of a tissue, a system, animal or human that is being sought by a researcher, veterinarian, medical doctor or other clinician. The term "prophylactically effective amount" is intended to mean that amount of a pharmaceutical drug that will prevent or reduce the risk of occurrence of the biological or medical event that is sought to be prevented in a tissue, a system, animal or human by a researcher, veterinarian, medical doctor or other clinician. Particularly, the dosage a patient receives can be selected so as to achieve the amount of LDL (low density lipoprotein) cholesterol lowering desired; the dosage a patient receives may also be titrated over time in order to reach a target LDL level. The dosage regimen utilizing the dihydroxy open acid statin of the present invention is selected in accordance with a variety of factors including type, species, age, weight, sex and medical condition of the patient; the severity of the condition to be treated; the potency of the compound chosen to be administered; the route of administration; and the renal and hepatic function of the patient. A consideration of these factors is well within the purview of the ordinarily skilled clinician for the purpose of determining the therapeutically effective or prophylactically effective dosage amount needed to prevent, counter, or arrest the progress of the condition.

The novel compounds of this invention are the crystalline hydrated forms of the calcium salt of dihydroxy open acid simvastatin. A particular crystalline hydrated calcium salt of dihydroxy open acid simvastatin is the one having an x-ray powder diffraction (XRPD) pattern obtained using CuKα radiation characterized by reflections at d-spacings of 30.7, 24.6, 15.9, 11.2, 8.58, 7.31, 6.74, 6.06, 5.35, 5.09, 4.93, 4.60, 3.93, 3.84, 3.07, 3.51 and 3.28Å. For convenience, the crystalline hydrated form of the calcium salt of dihydroxy open acid simvastatin having the above-defined XRPD pattern will also be referred to herein as compound I. Compound I can be represented two-dimensionally as a hydrated form of the following structural formula Ia:

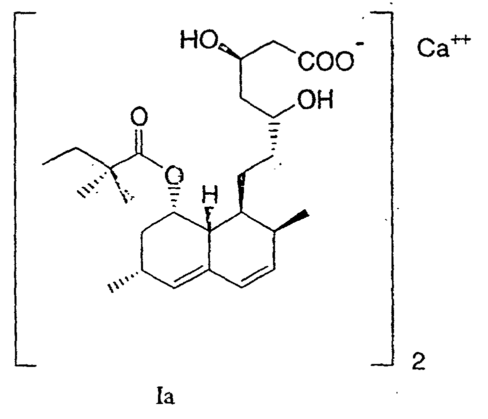

In addition to the XRPD pattern described above, compound I of the instant invention is further characterized by the thermogravimetry (TG) curve shown in Figure I. The TG curve for compound I was obtained under a nitrogen flow at a heating rate of 10°C/minute and is characterized by a 6.3% weight loss from ambient room temperature to a stable weight loss plateau at about 175°C. Additional weight losses due to the onset of decomposition of the compound are observed above about 190°C.

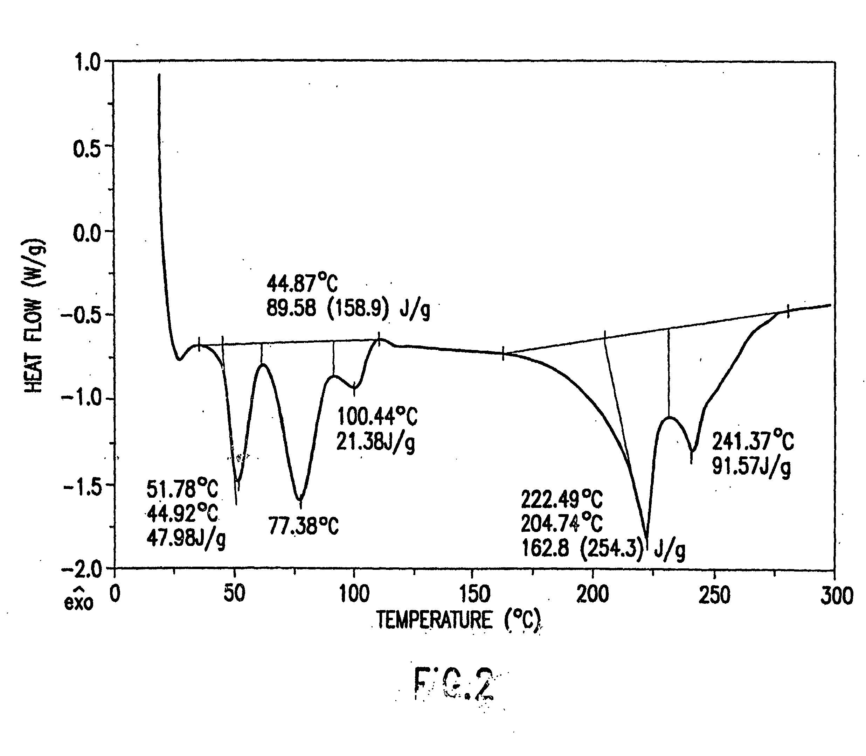

Compound I is still further characterized by the differential scanning calorimetry (DSC) curve shown in Figure 2. The DSC curve for compound I was obtained under a nitrogen flow bubbled through 16.0°C water at a heating rate of 10°C/minute in an open cup, and is characterized by three lower temperature endotherms with peak temperatures of 52, 77 and 100°C and associated heats of 48, 90 and 21 J/g, respectively, and two higher temperature endotherms due to decomposition with peak temperatures of 222 and 241°C and associated heats of 163 and 92 J/g.

Compound I of the instant invention is still further characterized by the ¹H nuclear magnetic resonance (NMR) spectral data, ¹³C NMR and mass spectral (MS) data as given in Example 1 herein.

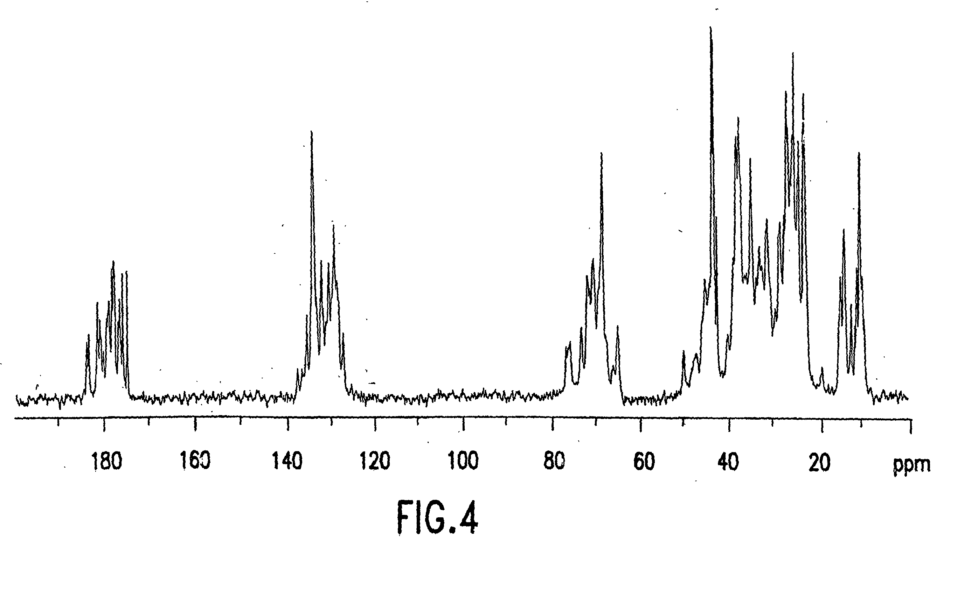

Compound I is also characterized by the solid-state ¹³C nuclear magnetic resonance spectrum shown in Figure 4, which was completed using a Bruker DSX 400WB NMR system operating at 100.6 MHz for ¹³C and 400.1 MHz for ¹H using a Bruker MAS 400WB BL7 double-resonance probe with a spinning module housing a 7 mm zirconia rotor with Kel-f end caps. The solid-state ¹³C nuclear magnetic resonance (NMR) spectrum was acquired using cross polarization (CP), magic-angle spinning (MAS), and high-power (∼59 kHz) decoupling with variable-amplitude cross-polarization and total sideband suppression. Proton and carbon 90° pulse widths were 4.25 µsec with a contact time of 2.0 msec. The sample was spun at 7.0 kHz and a total of 1024 scans were collected for the spectrum with a recycle delay of 3.0 seconds. A line broadening of 10 Hz was applied to the spectrum before FT was performed. Chemical shifts are reported on the TMS scale using the carbonyl carbon of glycine (176.03 ppm) as a secondary reference.

The compounds in accordance with the present invention can be used for the preparation of a medicament for inhibiting HMG-CoA reductase, and for treating lipid disorders including hypercholesterolemia, hypertriglyceridemia and combined hyperlipidemia. They are also useful for preventing or reducing the risk of developing atherosclerosis, as well as for halting or slowing the progression of atherosclerotic disease once it has become clinically evident, in a patient who is at risk of developing atherosclerosis or who already has atherosclerotic disease.

Atherosclerosis encompasses vascular diseases and conditions that are recognized and understood by physicians practicing in the relevant fields of medicine. Atherosclerotic cardiovascular disease including restenosis following revascularization procedures, coronary heart disease (also known as coronary artery disease or ischemic heart disease), cerebrovascular disease including multi-infarct dementia, and peripheral vessel disease including erectile dysfunction are all clinical manifestations of atherosclerosis and are therefore encompassed by the terms "atherosclerosis" and "atherosclerotic disease".

The statin according to the present invention may be administered to prevent or reduce the risk of occurrence, or recurrence where the potential exists, of a coronary heart disease event, a cerebrovascular event, and/or intermittent claudication. Coronary heart disease events are intended to include CHD death, myocardial infarction (i.e., a heart attack), and coronary revascularization procedures. Cerebrovascular events are intended to include ischemic or hemorrhagic stroke (also known as cerebrovascular accidents) and transient ischemic attacks. Intermittent claudication is a clinical manifestation of peripheral vessel disease. The term "atherosclerotic disease event" as used herein is intended to encompass coronary heart disease events, cerebrovascular events, and intermittent claudication. It is intended that persons who have previously experienced one or more non-fatal atherosclerotic disease events are those for whom the potential for recurrence of such an event exists.

Accordingly, the statin according to the present invention can also be used for the preparation of a medicament for preventing or reducing the risk of a first or subsequent occurrence of an atherosclerotic disease event in a patient at risk for such an event. The patient may or may not have atherosclerotic disease at the time of administration, or may be at risk for developing it.

The statin of the instant invention is also useful for preventing and/or treating inflammatory diseases or disorders alone or in conjunction with the treatment of conditions described above. This includes, for example, the treatment of inflammatory conditions susceptible to treatment with a non-steroidal anti-inflammatory agent, arthritis including rheumatoid arthritis, and degenerative joint diseases (osteoarthritis), dementia, Alzheimer's disease, multiple sclerosis, inflammatory bowel disease, asthma, psoriasis, systemic lupus erythematosis, vasculitis, gout, adrenoleukodystrophy, diabetic retinopathy, nephropathy and diabetes mellitus type II.

Persons requiring treatment with the statin of the present invention include those at risk of developing atherosclerotic disease and of having an atherosclerotic disease event. Standard atherosclerotic disease risk factors are known to the average physician practicing in the relevant fields of medicine. Such known risk factors include but are not limited to hypertension, smoking, diabetes, low levels of high density lipoprotein (HDL) cholesterol, and a family history of atherosclerotic cardiovascular disease. Published guidelines for determining those who are at risk of developing atherosclerotic disease can be found in: National Cholesterol Education Program, Second report of the Expert Panel on Detection, Evaluation, and Treatment of High Blood Cholesterol in Adults (Adult Treatment Panel II), National Institute of Health, National Heart Lung and Blood Institute, NIH Publication No. 93-3095, September 1993; abbreviated version: Expert Panel on Detection, Evaluation, and Treatment of High Blood Cholesterol in Adults, Summary of the second report of the national cholesterol education program (NCEP) Expert Panel on Detection, Evaluation, and Treatment of High Blood Cholesterol in Adults (Adult Treatment Panel II), JAMA, 1993, 269, pp. 3015-23. People who are identified as having one or more of the above-noted risk factors are intended to be included in the group of people considered at risk for developing atherosclerotic disease. People identified as having one or more of the above-noted risk factors, as well as people who already have atherosclerosis, are intended to be included within the group of people considered to be at risk for having an atherosclerotic disease event.

The oral dosage amount of the crystalline hydrated form of the calcium salt of dihydroxy open acid simvastatin such as compound I is from about 5 to 200 mg/day, and more preferably from about 12.5 to 100 mg/day and most preferably 12.5 to 50 mg/day. However, dosage amounts will vary depending on factors as noted above, including the potency of the particular compound. Although the active drug of the present invention may be administered in divided doses, for example from one to four times daily, a single daily dose of the active drug is preferred. As examples, the daily dosage amount may be selected from, but not limited to, 5 mg, 10 mg, 12.5 mg, 15 mg, 20 mg, 25 mg, 40 mg, 50 mg, 75 mg, 80 mg, 100 mg, 150 mg and 200 mg.

The active drug employed in the instant therapy can be administered in such oral forms as tablets, capsules, pills, powders, granules, elixirs, tinctures, suspensions, syrups, and emulsions. Oral formulations are preferred.

For the crystalline hydrated forms of the calcium salt of dihydroxy open acid simvastatin, for example compound I, administration of the active drug can be via any pharmaceutically acceptable route and in any pharmaceutically acceptable dosage form. This includes the use of oral conventional rapid-release, time controlled-release and delayed-release (such as described above) pharmaceutical dosage forms. An oral delayed-release dosage formulation of the instant drug is preferred, and particularly an enteric overcoat surrounding a rapid-release dosage unit, or the GEM controlled-release drug delivery device with an enteric overcoat surrounding the dosage unit. Additional suitable pharmaceutical compositions for use with the present invention are known to those of ordinary skill in the pharmaceutical arts; for example, see Remington's Pharmaceutical Sciences, Mack Publishing Co., Easton, PA.

In the compositions of the present invention, the active drug is typically present in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with a non-toxic, pharmaceutically acceptable, inert carrier such as lactose, starch, sucrose, glucose, modified sugars, modified starches, methyl cellulose and its derivatives, dicalcium phosphate, calcium sulfate, mannitol, sorbitol and other reducing and non-reducing sugars, magnesium stearate, stearic acid, sodium stearyl fumarate, glyceryl behenate, calcium stearate and the like. For oral administration in liquid form, the drug components can be combined with non-toxic, pharmaceutically acceptable inert carriers such as ethanol, glycerol, water and the like. Moreover, when desired or necessary, suitable binders, lubricants, disintegrating agents and coloring and flavoring agents can also be incorporated into the mixture. Stabilizing agents such as antioxidants, for example butylated hydroxyanisole (BHA), 2,6-di-tert-butyl-4-methylphenol (BHT), propyl gallate, sodium ascorbate, citric acid, calcium metabisulphite, hydroquinone, and 7-hydroxycoumarin, particularly BHA, propyl gallate and combinations thereof, can also be added to stabilize the dosage forms; the use of at least one stabilizing agent is preferred with the instant composition. Preferably an antioxidant is employed with the crystalline hydrated form of the calcium salt of dihydroxy open acid simvastatin, and particularly compound I. Other suitable components include gelatin, sweeteners, natural and synthetic gums such as acacia, tragacanth or alginates, carboxymethylcellulose, polyethylene glycol, waxes and the like.

The active drug can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Active drug may also be delivered by the use of monoclonal antibodies as individual carriers to which the compound molecules are coupled. Active drug may also be coupled with soluble polymers as targetable drug carriers. Such polymers can include polyvinyl-pyrrolidone, pyran copolymer, polyhydroxy-propyl-methacrylamide-phenol, polyhydroxy-ethyl-aspartamide-phenol, or polyethyleneoxide-polylysine substituted with palmitoyl residues. Furthermore, active drug may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphipathic block copolymers of hydrogels.

The instant invention also encompasses a process for preparing a pharmaceutical composition comprising combining a crystalline hydrated form of the calcium salt of dihydroxy open acid simvastatin with a pharmaceutically acceptable carrier. Also encompassed is the pharmaceutical composition which is made by combining a crystalline hydrated form of the calcium salt of dihydroxy open acid simvastatin with a pharmaceutically acceptable carrier.

In a broad embodiment, any suitable additional active agent or agents may be used in combination with the dihydroxy open acid statin in a single dosage formulation, or may be administered to the patient in a separate dosage formulation, which allows for concurrent or sequential administration of the active agents. One or more additional active agents may be administered with the dihydroxy open acid statin. The additional active agent or agents can be lipid lowering compounds or agents having other pharmaceutical activities, or agents that have both lipid-lowering effects and other pharmaceutical activities. Examples of additional active agents which may be employed include but are not limited to HMG-CoA synthase inhibitors; squalene epoxidase inhibitors; squalene synthetase inhibitors (also known as squalene synthase inhibitors); acyl-coenzyme A:cholesterol acyltransferase (ACAT) inhibitors including selective inhibitors of ACAT-1 or ACAT-2 as well as dual inhibitors of ACAT-1 and 2; microsomal triglyceride transfer protein (MTP) inhibitors; probucol; niacin; cholesterol absorption inhibitors such as SCH-58235; bile acid sequestrants; LDL (low density lipoprotein) receptor inducers; platelet aggregation inhibitors, for example glycoprotein IIb/IIIa fibrinogen receptor antagonists and aspirin; human peroxisome proliferator activated receptor gamma (PPARγ) agonists including the compounds commonly referred to as glitazones for example troglitazone, pioglitazone and rosiglitazone, and including those compounds included within the structural class known as thiazolidinediones as well as those PPARγ agonists outside the thiazolidinedione structural class; PPARα agonists such as clofibrate, fenofibrate including micronized fenofibrate, and gemfibrozil; PPAR dual α/γ agonists; vitamin B₆ (also known as pyridoxine) and the pharmaceutically acceptable salts thereof such as the HCl salt; vitamin B₁₂ (also known as cyanocobalamin); folic acid or a pharmaceutically acceptable salt or ester thereof such as the sodium salt and the methylglucamine salt; anti-oxidant vitamins such as vitamin C and E and beta carotene; beta-blockers; angiotensin II antagonists such as losartan; angiotensin converting enzyme inhibitors such as enalapril and captopril; calcium channel blockers such as nifedipine and diltiazem; endothelin antagonists; agents that enhance ABC1 gene expression; FXR and LXR ligands including both inhibitors and agonists; bisphosphonate compounds such as alendronate sodium; and cyclooxygenase-2 inhibitors such as rofecoxib and celecoxib. Additionally, the dihydroxy open acid statins of this invention, for example compound I, may be used in combination with anti-retroviral therapy in AIDS infected patients to treat lipid abnormalities associated with such treatment or with AIDS itself, for example, but not limited to, their use in combination with HIV protease inhibitors such as indinavir, nelfinavir, ritonavir and saquinavir.

A therapeutically or prophylactically effective amount, as appropriate, of a crystalline hydrated form of the calcium salt of dihydroxy open acid simvastatin, for example compound I, can be used for the preparation of a medicament useful for inhibiting HMG-CoA reductase, as well as for treating and/or reducing the risk for diseases and conditions affected by inhibition of HMG-CoA reductase, such as treating lipid disorders, preventing or reducing the risk of developing atherosclerotic disease, halting or slowing the progression of atherosclerotic disease once it has become clinically manifest, and preventing or reducing the risk of a first or subsequent occurrence of an atherosclerotic disease event. For example, the medicament may be comprised of about 5 mg to 200 mg of a crystalline hydrated form of dihydroxy open acid simvastatin, or more particularly about 12.5 mg to 100 mg, and most particularly 12.5 to 50 mg.

A therapeutically or prophylactically effective amount, as appropriate, of a crystalline hydrated form of the calcium salt of dihydroxy open acid simvastatin, for example compound I, can be used for the preparation of an oral medicament adapted for delayed-release, wherein substantial release of the statin after oral administration does not occur until after passage of the medicament through the stomach, or alternately wherein at least 90%, particularly at least 95% and more particularly at least 99%, of the statin is delivered in its dihydroxy open acid form to the intestinal mucosa of a patient after oral administration, as measured by the geometric mean ratio of the plasma AUC (area under the curve) of active vs. total HMG-CoA reductase inhibitory activity as described above. Said oral medicaments are also useful for inhibiting HMG-CoA reductase, as well as for treating and/or reducing the risk for diseases and conditions affected by inhibition of HMG-CoA reductase, as described above.

The medicament comprised of a dihydroxy open acid statin, for example compound I, may also be prepared with one or more additional active agents, such as those described supra.

As noted above, simvastatin is a semi-synthetic product which can be made from the natural product lovastatin. Processes for preparing lovastatin and simvastatin are well documented in the published literature. For example, U.S. Patent No. 4,231,938 describes a fermentation and isolation process for obtaining lovastatin using the microorganism *Aspergillus terreus.* U.S. Patent No.'s 4,444,784, 4,820,850, 4,916,239 and 4,582,915 describe methods for making dihydroxy open-acid and lactonized forms of simvastatin.

In a further aspect, the present invention provides a process for preparing the compounds in accordance with the invention, which process comprises the steps of:
A) combining a mixture of a salt of dihydroxy open acid simvastatin in an aqueous solvent with calcium acetate hydrate to form an amorphous calcium salt of dihydroxy open acid simvastatin, wherein the aqueous solvent is selected from water, an aqueous-protic organic solvent mixture and an aqueous-aprotic organic solvent mixture;
B) aging the resulting mixture at a temperature up to 50°C until turnover of the amorphous calcium salt of dihydroxy open acid simvastatin to the crystalline calcium salt of dihydroxy open acid simvastatin is complete;
C) recovering the crystalline calcium salt of dihydroxy open acid simvastatin; and
D) drying the recovered crystals under a moist atmosphere.

Suitable salts for use in step (A) of the above process include those which are generally prepared by reacting the free acid with a suitable organic or inorganic base. The process of salt exchange from any salt to the calcium salt is feasible if the starting salt is more soluble in the reaction solvent than the calcium salt. Examples of suitable salts may include, but are not limited to, those formed from cations such as sodium, potassium, aluminum, lithium, and tetramethylammonium, as well as those salts formed from amines such as ammonia, ethylenediamine, N-methylglucamine, lysine, arginine, ornithine, choline, N,N'-dibenzylethylenediamine, chloroprocaine, diethanolamine, procaine, N-benzylphenethylamine, 1-p-chlorobenzyl-2-pyrrolidin-1'-yl-methylbenzimidazole, diethylamine, piperazine, morpholine, 2,4,4-trimethyl-2-pentamine and tris(hydroxymethyl)-aminomethane.

Simvastatin and its dihydroxy open acid counterpart, including compound I, tend to form oxidative by-products; therefore, to minimize the formation of such by-products, it is preferred that the procedures used to make compound I are performed under an inert atmosphere such as nitrogen. Although compound I can be obtained without using an inert atmosphere, the purity of the desired product will not be optimized.

Hydrolysis of the lactone ring of simvastatin can be accomplished by treating simvastatin with at least one equivalent, and preferably a slight excess of one equivalent, of an aqueous base. If more than a slight excess of base is used, the excess base is preferably neutralized before proceeding to the salt formation step in order to prevent formation of insoluble calcium hydroxide or calcium carbonate by-product. The base employed for the hydrolysis can be an aqueous solution of a metal hydroxide or metal carbonate, for example but not limited to sodium hydroxide, potassium hydroxide, lithium hydroxide, sodium carbonate and potassium carbonate. Sodium hydroxide is preferred. The hydrolysis can be performed in water, an aqueous-protic organic solvent mixture, or an aqueous-aprotic organic solvent mixture. Suitable protic organic solvents include but are not limited to methanol (MeOH), ethanol (EtOH), isopropyl alcohol, and n-propyl alcohol (propanol). Examples of suitable aprotic organic solvents include but are not limited to acetonitrile, dimethyl sulfoxide (DMSO), N,N-dimethylformamide (DMF), tetrahydrofuran (THF), *tert*-butyl methyl ether (MTBE) and toluene. Particularly, an aqueous ethanol or n-propyl alcohol solvent mixture may be used, and more particularly an aqueous n-propyl alcohol solvent mixture.

After the hydrolysis reaction is complete, the pH of the reaction mixture is adjusted to about 6 to 11, particularly 6 to 9, and more particularly 7 to 8.5, by addition of an acid. In this pH range, the dihydroxy open acid simvastatin will exist as a metal salt, for example as the sodium salt if the base used in the hydrolysis step is sodium hydroxide or sodium carbonate. Any acid that is capable of forming a soluble calcium salt such as calcium chloride or calcium citrate, is suitable. A soluble calcium salt is intended to be a salt that is soluble in the solvent system employed in the instant process. Preferably an acid such as acetic acid (HOAc) or a mineral acid is employed, particularly HCl.

The resulting pH-adjusted reaction mixture containing the metal salt of dihydroxy open acid simvastatin is next combined with a solution of about 0.50 to 0.55 equivalents of calcium acetate hydrate [Ca(OAc)₂•xH₂O] in water or an aqueous-protic organic solvent mixture, such as aqueous EtOH, i-PrOH, n-PrOH, acetonitrile, MeOH, DMF, DMSO, THF, and particularly aqueous EtOH or aqueous n-propyl alcohol. The pH-adjusted reaction mixture can be added to the calcium acetate hydrate solution, or the calcium acetate hydrate solution can be added to the pH-adjusted reaction mixture. The addition can occur all at once, or optionally it can be performed in portions over time with periods of aging. For example, a small portion, e.g., about one-quarter, of the calcium acetate hydrate solution can be added to the pH-adjusted reaction mixture over a short period of time, for example over about 30 minutes, and then the resulting mixture can be allowed to age for an additional short period of time at room temperature, optionally followed by a further period of aging at a temperature up to about 50°C, for example from about 10°C up to about 50°C, particularly from room temperature up to about 50°C, more particularly from about 30 to 40°C, and most particularly from about 30 to 35°C, after which the remaining calcium acetate hydrate solution can be added in portions over several hours at a temperature up to about 50°C, for example from about 10°C up to about 50°C, particularly from room temperature up to about 50°C, and more particularly from about 30 to 40°C and most particularly from about 30 to 35°C. Optionally, the reaction mixture can be seeded with crystalline Compound I.

Whether the pH-adjusted reaction mixture and the calcium acetate hydrate solution are combined at once or in portions, the resulting slurry must be aged until turnover of the resulting amorphous calcium salt of dihydroxy open acid simvastatin to the crystalline product is complete, usually for at least several hours. Complete turnover to the crystalline' product can be assessed by standard techniques in the art, for example, by examining a sample of the product under a microscope. This aging step can be performed at a temperature up to about 50°C, for example from about 10°C up to about 50°C, particularly from room temperature up to about 50°C, and more particularly from about 30 to 40°C and most particularly from about 30 to 35°C. During the aging period or periods, the use of lower temperatures will lead to crystallized product; however, it has been found that as the temperature drops, the rate of crystal turnover becomes slower, making the procedure less time-efficient.

If necessary, the slurry is then allowed to cool to room temperature and is collected by suction filtration. The recovered solid is suction dried under a moist atmosphere (about 30 to 70% relative humidity, particularly 40 to 70%), preferably a moist inert atmosphere such as nitrogen and particularly at a temperature from about 10 to 40°C, and more particularly 25 to 35°C. The final step of suction filtration in the recovery of compound I should be done under a moist atmosphere, and preferably a moist inert atmosphere in order to minimize oxidative by-products. If an additional step of adding an antioxidant to compound I is performed, as described below, then the final suction filtration is the one performed after combining the antioxidant with compound I.

As noted above, compound I has a tendency to oxidize upon contact with air, and one way to minimize oxidation is to perform the reaction sequence under an inert atmosphere. Additionally, one or more anti-oxidants such as BHA, BHT, propyl gallate, ascorbic acid, calcium metabisulphite, hydroquinone, nordihydroguaiaracetic acid (NDGA) or 7-hydroxycoumarin can be combined with compound I. This is done by agitating a slurry of compound I with the antioxidant and recovering the resulting solid by suction filtration.

Alternatively, the ammonium salt of dihydroxy open acid simvastatin can be used as the starting material to be combined with the calcium acetate hydrate, thus avoiding the hydrolysis and pH adjustment steps needed when starting with lactonized simvastatin. The other reaction conditions described above, such as solvents, temperatures, etc., can otherwise be employed.

The following non-limiting Examples are intended to illustrate the present invention.

Abbreviations which may appear herein are as follows: MeOH is methanol; EtOH is ethanol; PrOH is propanol; HOAc is acetic acid; MeCN is acetonitrile; DMSO is dimethyl sulfoxide; DMF is N,N-dimethylformamide; Ca(OAc)₂ is calcium acetate; HPLC is high performance liquid chromatography; min. is minutes; h is hour(s); D.I. is de-ionized; NMR is nuclear magnetic resonance; EI MS is electron impact mass spectrum; HR-EI MS is high resolution electron impact mass spectrum; RH is relative humidity. The "seed" used in the examples is Compound I.

### EXAMPLE 1

Preparation of crystalline hydrated calcium salt of dihydroxy open acid simvastatin (Compound I)

A 22 L four-necked round bottom flask was equipped with a temperature probe, a N₂ inlet, an addition funnel, and an overhead stirrer. 8.0 L of 15% EtOH-H₂O was added and the solution was purged with N₂ for 10 min. Simvastatin (396 g, 0.946 mol) was added, and the slurry was purged with N₂ for 5 min. Then 5N NaOH (198 mL) was added at room temperature. After about 1 hour, the hydrolysis reaction was complete as analyzed by HPLC (>99.9% conversion). The pH of the reaction solution was adjusted to 7 to 8.5 by addition of 1 N HCl (approx. 65 mL). A solution of Ca(OAc)₂•H₂O (116.6 g, 0.662 mol) in 4.0 L of 60% EtOH-H₂O was purged with nitrogen for 5 min. A 1.0 L portion of this solution was added to solution of the sodium salt over 30 min. The resulting slurry was aged at room temperature for 30 min, and then at 30 to 35°C for 1-2 h.

The rest of Ca(OAc)₂ in EtOH-water was added over approx. 30 min at 30 to 35°C. The slurry was aged at 30 to 35°C for 5 hours under atmosphere of N₂. The slurry was cooled to room temperature and was collected by suction-filtration. The wet cake was washed with 4 L 30% EtOH-H₂O, 4 L 20% EtOH-H₂O, followed by 6 L x 3 of D.I. water. The solid was suction dried under an atmosphere of moist N₂ (40 to 70% relative humidity) at room temperature for 4 days. Crystalline hydrated calcium salt of dihydroxy open acid simvastatin was obtained as a white powder.

The calcium salt was delumped with a single pass through a cleaned QUADRO™ COMIL® (Model 197S).

| HPLC CONDITIONS | |
|---|---|
| Column | YMC Basic 4.6 mm x 25 cm |
| Detector | ABS 757 1AU/volt output |
| Sample solvent | EtOH/CH₃CN/H₂O (1:1:1) |
| Column temp | 25°C |
| Flow rate | 1.5 mL / min. |
| Wavelength | 238 and 210 nm |

| Gradient: | | %H₂O (10 mM K₂HPO₄₋ |
|---|---|---|
| Time (mm.) | %CH₃CN | KH₂PO₄, pH = 6.5) |
| 0.00 | 30 | 70 |
| 20.00 | 45 | 55 |
| 34.00 | 70 | 30 |
| 39.00 | 70 | 30 |
| 39.50 | 30 | 70 |
| 43.00 | 30 | 70 |

| | |
|---|---|
| Retention time of simvastatin open acid: | 17.07 min. |
| Retention time of simvastatin: | 32.90 min. |

### SPECTRAL DATA

¹H NMR (400 MHz, CD₃OD), δ 5.97 (d, J = 9.6 Hz, 1 H), 5.77 (dd, J = 9.6, 5.2 Hz, 1 H), 5.49 (m, 1 H), 5.33 (m, 1 H), 4.17 (m, 1 H), 3.70 (m, 1 H), 2.44 - 2.35 (m, 2 H), 2.42 (dd, J = 15.7, 3.6 Hz, 1 H), 2.31 - 2.27 (m, 1 H), 2.29 (dd, J = 15.7, 8.4 Hz, 1 H), 2.00 (ddd, J = 15.7, 7.6, 2.4 Hz, 1 H), 1.93 (m, 1 H), 1.68 (m, 1 H), 1.61 - 1.55 (m, 2 H), 1.55 (m, 2 H), 1.42 (m, 1 H), 1.32 (m, 1 H), 1.19 (m, 1 H), 1.12 (s, 6 H), 1.08 (d, J = 7.2 Hz, 3 H), 0.89 (d, J = 7.2 Hz, 3 H), 0.84 (t, J = 7.6 Hz, 3 H) ppm.
¹³C NMR (100.55 MHz, CD₃OD), δ 182.3, 179.3, 134.1, 133.2, 130.3, 129.6, 72.5, 69.8, 45.1, 44.4, 44.1, 38.8, 38.3, 36.4, 34.3, 33.9, 32.0, 28.6, 25.9, 25.37, 25.36, 23.7, 14.3, 9.9 ppm
EI MS: m/e: 437 (M+H), 419(M+H-H₂O), 303.
HR-EI MS: Calcd. for C₂₅H₃₈O₅ 418.2719; Found: 418.2712.

### EXAMPLE 2

### Preparation of crystalline hydrated calcium salt of dihydroxy open acid simyastatin (Compound I) with BHA

A 22 L three-necked round bottom flask was equipped with a temperature probe, a N₂ inlet, an additional funnel, and an overhead stirrer. 8.0 L of 15% EtOH-H₂O was added and purged with N₂ for 10 min. Simvastatin (396 g, 0.946 mol) was added, and the slurry purged with N₂ for 5 min. 198 mL of 5N NaOH (0.993 mol, 1.05 equiv.) was then added at room temperature. The hydrolysis reaction is usually complete in 1 h. as analyzed by HPLC (> 99.9% conversion). The pH of the reaction solution was adjusted to 7 to 8.5 by addition of 1 N HCl (about 65 mL).

A solution of Ca(OAc)₂•H₂O (91.7 g, 0.520 mol, 0.55 equiv.) in 4.0 L of 60% EtOH- H₂O was purged with nitrogen for 5 min. 1.0 L of this solution was added to reaction solution over 30 min. The slurry was aged at room temperature for 30 min, and then at 30 to 35°C for 1-2 h. The rest of the Ca(OAc)₂ in EtOH-water was added in portions over 3h hours at 30-35°C. The slurry was allowed to age at 30 to 35°C for 5 h under an atmosphere of N₂. The slurry was allowed to cool to room temperature and was collected by suction-filtration. The wet cake was washed with 4 L 30% EtOH-H₂O, 4 L 20% EtOH-H₂O, followed by 6 L x 3 of D.I. water. The solid was suction dried under atmosphere of moist N₂ (40 to 70% RH) at room temperature to give 1.7 Kg of wet cake.

The above wet cake was placed in a clean 20 L three necked flask under atmosphere of nitrogen. A solution of BHA (2.603 g, 0.2 wt % equiv) in degassed 15% EtOH H₂O (8.5 L) was added, followed by addition of degassed water (2.55 L), and the slurry was agitated at room temperature for 1 to 2 h. The solid was collected by suction filtration under atmosphere of moist N₂ with no washing to give 1.49 Kg wet cake. The solid was suction dried under atmosphere of moist N₂ (40 to 70% RH) at room temperature for 4 days. The calcium salt title product was obtained as a white powder (94% yield. 99.4%A at 238 nm, 0.2wt% BHA, KF = 73 %wt).

### EXAMPLE 3

### Preparation of crystalline hydrated calcium salt of dihydroxy oper acid simvastatin (Compound I) in aqueous nPrOH: Conventional addition mode

### Step 1: Hydrolysis

A 72 L three-necked round bottom flask was equipped with a temperature probe, a N₂ inlet, an additional funnel, and an overhead stirrer. 28.5 L of D.I. H₂O was added and purged with N₂ for 10 min. 1.5 Kg simvastatin was added, followed by 788mL of 5N NaOH in one portion at room temperature. The hydrolysis reaction is usually complete in 2 h. as analyzed by HPLC (>99.9% conversion). 1.5L of nPrOH was added and the pH of the reaction solution was adjusted to 9.5 to 11.0 by adding 2 N HOAc (about 170 mL).

### Step 2: Salt Formation

150 g of seed (Compound I) was added to the above solution and the resulting slurry was allowed to warm up to 35 to 40 °C. A solution of Ca(OAc)₂•H₂O (347 g) in 15 L of 20% nPrOH was purged with nitrogen for 5min. and added to slurry over 3 h. The resulting slurry was aged at 35 to 40 °C for 5 h. under an atmosphere of N₂ and then cooled to room temperature. The solid was collected by filtration and was washed with 10% nPrOH-H₂O (15 L x 3).

### Step 3: BHA loading

The above wet cake (9.1 kg) was transferred into a clean 72 L three necked flask under an atmosphere of nitrogen. A solution of BHA (7.6 g) in-degassed 10% nPrOH (45 L) was added and the slurry was agitated at room temperature for 1 h. and filtered under atmosphere of N₂, and then suction dried under atmosphere of moist N₂ (30 to 70% RH) at room temperature for 7 days. 1.78 Kg of Ca salt title product was obtained as a white powder (94% yield. 99.4%A at 238 nm, 0.2wt% BHA, KF = 6.6 %wt).

### EXAMPLE 4

### Preparation of hydrated crystalline calcium salt of dihydroxy open acid simvastatin in aqueous PrOH: Simultaneous addition mode

The process described in this example allows for keeping half of the batch in the vessel at all times as seed in a semi-continuous process.

100 g of simvastatin was hydrolyzed in 1.9 L water as described in Example 3. Then, 100 ml nPrOH was added and the solution pH was adjusted to 9 to 11 with 1 N HOAc. The resulting solution and a solution of Ca(OAc)₂•H₂O (23.2 g) in 1.0 L of 20% nPrOH were added separately but simultaneously to a suspension of 10-50 wt% Ca salt in 10% nPrOH (30 volume 10% PrOH relative to the amount of the seed) at 30 to 40°C over 3 h. After 5 h age at 30 to 40°C, the slurry was cooled to room temperature, filtered, and loaded with anti-oxidant and dried as described in conventional addition mode process. 95% yield.

### EXAMPLE 5

### Preparation of crystalline hydrated calcium salt of dihydroxy open acid simvastatin (Compound I) in aqueous PrOH: Loading BHA through co-crystallization

Method A: 100 g of simvastatin was hydrolyzed in 1.9 L water as described in Example 3. Then, 100 ml nPrOH was added arid the solution pH was adjusted to 9 to 11 with 1 N HOAc. 10wt% seed was added and the slurry was allowed to warm up to 35 to 40°C. A solution of Ca(OAc)₂•H₂O (23.2 g) and BHA (540 mg) in 1.0 L of 20% nPrOH were added to the slurry at 35 to 40°C over 3 h. After 5 h age at 30 to 40°C, the slurry was cooled to room temperature, filtered and washed with a solution of BHA (0.1g/L) in 10% nPrOH (1L x 3). The wet cake was dried under moist N₂ as described in conventional addition mode process. The final dried Ca salt title product contained 0.2wt% BHA. 95% yield.

Method B: The procedure of Method A was employed with the following change. Instead of adding BHA to a Ca(OAc)₂ solution, the same amount of BHA was added into the pH adjusted solution of hydrolyzed simvastatin at room temperature. The solution was warmed to 35 to 40°C to dissolve BHA. Then, 10wt% seed was introduced. The rest of the steps were as described in Method A. The final dried Ca salt title product contained 0.2wt% BHA. 95% yield.

### EXAMPLE 6

### Preparation of crystalline hydrated calcium salt of dihydroxy open acid simvastatin (Compound I) in aqueous PrOH: Loading BHA/propyl gallate

Starting with 2.0 Kg of simvastatin, the calcium salt of dihydroxy open acid simvastatin was crystallized, isolated, and washed as described in Example 3. The first wet cake was transferred to a clean 100L vessel under atmosphere of N₂. A solution of BHA (9.2g) and propyl gallate (11.2 g) in 50 L 10% nPrOH was added to above vessel. The slurry was aged at room temperature for 1h. The slurry was filtered with no wash. The wet cake was dried under moist N₂. 95% yield. The dried salt was loaded with 0.07wt% propyl gallate and 0.2 wt% BHA.

### EXAMPLE 7

### Preparation of crystalline hydrated calcium salt of dihydroxy open acid simyastatin (Compound I) in aqueous PrOH: Loading propyl gallate

Starting with 2.0 Kg of simvastatin, the calcium salt of dihydroxy open acid simvastatin was crystallized, isolated, and washed as described in Example 3. Then, the wet cake was washed with 10L of a solution of propyl gallate in 10% nPrOH (propyl gallate concentration = 0.224 g/L). Then, 20 L of propyl gallate solution in 10% nPrOH (propyl gallate concentration = 0.224 g/L) was added and the wet cake was mixed in the filtration pot before filtration. The wet cake was dried under moist N₂. 95% yield. The dried salt was loaded with 0.07wt% propyl gallate.

### EXAMPLE 8

### Preparation of crystalline hydrated calcium salt of dihydroxy open acid simvastatin (Compound I) Loading BHA, BHA/Vitamin E, and Vitamin E in heptane

100 g of simvastatin was hydrolyzed in 1.9 L water as described in Example 3. Then, 100 ml nPrOH was added and the solution pH was adjusted to 9 to 11 with 1 N HOAc. 10wt% seed was added and the slurry was allowed to warm up to 35 to 40°C. A solution of Ca(OAc)₂•H₂O (23.2 g) in 1.0 L of 20% nPrOH was added to a slurry at 35 to 40°C over 3 h. After 5 h age at 30 to 40°C, the slurry was cooled to room temperature. The calcium salt slurry was filtered and washed with 10% nPrOH (500 mL x 1), followed by water (1L x 3). The wet cake (KF = 75 to 80 wt% water) was then washed with 1 L of heptane, to displace most of the water. This wet cake was washed with a solution of BHA or Vitamin E or BHA/Vitamin E (conc. = 1.38 g/ L, 800 mL) and dried under moist N₂.

### EXAMPLE 9

### Preparation of crystalline hydrated calcium salt of dihydroxy open acid simyastatin (Compound I) in aqueous MeCN

A 7.2 L three-necked round bottom flask was equipped with a temperature probe, a N₂ inlet, an additional funnel, and an overhead stirrer. 2.1 L of D.I. H₂O was added and purged with N₂ for 10 min. 150 g simvastatin was added, followed by 78.8mL of 5N NaOH in one portion at room temperature. The hydrolysis reaction is usually complete in 2 h. as analyzed by HPLC (> 99.9% conversion). 900 mL of MeCN was added and the pH of the reaction solution was adjusted to 9.5 to 11.0 by adding 2 N HOAc (about 17 mL).

30.0 g crystalline seed was added to above solution and the resulting slurry was allowed to warm up to 30 to 35°C. A solution of Ca(OAc)₂•H₂O (34.7 g) in 1.5 L of 30% MeCN was purged with nitrogen for 5 min. and added to reaction slurry over 3 h. The slurry was at 35 to 40°C for 5 h. under atmosphere of N₂. The slurry was allowed to cool to room temperature and the solid was collected by filtration. The wet cake was washed with 30% MeCN (1.5 L) and 10% MeCN (1.0 L), and rinsed/washed with a solution of BHA (0.9 g/L) in degassed 10% MeCN (1.0 L x 2. The solid was suction dried under atmosphere of moist N₂ (30 to 70% RH) at room temperature for 5 days. 1.67 Kg of the title compound was obtained as a white powder (88% yield. 99.4%A at 238 nm, 0.2wt% BHA, KF = 6.6 %wt).

### EXAMPLE 10

### Preparation of crystalline hydrated calcium salt of dihydroxy open acid simvastatin (Compound I) in aqueous MeOH

50 g simvastatin was hydrolyzed in 850 mL water as described in Example 3. Then, 150 ml MeOH was added and the solution pH was adjusted to 7 to 11 with 1N HOAc. 10wt% seed was added and the slurry was allowed to warm up to 30-35°C. A solution of Ca(OAc)₂•H₂O (11.6 g) in 500 mL of 30% MeOH was added to the slurry at 30-35°C over 3 h. After 5 h age at 30-35°C, the slurry was cooled to room temperature. The dihydroxy open acid simvastatin calcium salt slurry was filtered and washed with 20% MeOH (200 ml) and water (500 ml x 3). The wet cake was dried under moist N₂. The final dried Ca salt title product was isolated in 96% yield.

### EXAMPLE 11

### Preparation of crystalline hydrated calcium salt of dihydroxy open acid simvastatin (Compound I) in aqueous i-PrOH, DMF, DMSO

50 g simvastatin was hydrolyzed in 850 mL water as described in Example 3. Then, 150 ml i-PrOH was added and the solution pH was adjusted to 7 to 11 with 1 N HOAc. 10wt% seed was added and the slurry was allowed to warm up to 30-35°C. A solution of Ca(OAc)₂•H₂O (11.6 g) in 500 mL of 30% i-PrOH was added to the slurry at 30-35°C over 3 h. After 5 h age at 30-35°C, the slurry was cooled to room temperature. Ca salt slurry was filtered and washed with 20% ml i-PrOH (200 ml) and with water (500 ml x 3). The wet cake was dried under moist N₂. The final dried Ca salt title product was isolated in 96% yield.

The same procedure could be applied to prepare Compound I in DMF, DMSO, and similar solvents.

### EXAMPLE 12

### Preparation of crystalline hydrated calcium salt of dihydroxy open acid simyastatin (Compound I) in water

50 g of simvastatin was hydrolyzed in 1.0 L water as described in Example 3. Then, the solution pH was adjusted to 11 with 1 N HOAc. 10wt% seed was added and the slurry was allowed to warm up to 35-40°C. A solution of Ca(OAc)₂•H₂O (11.6 g) in 500 mL of water was added to the slurry at 35-40°C over 5 h. After 10 h age at 35-40°C, the slurry was cooled to room temperature. Ca salt slurry was filtered and washed with water (500 ml x 3). The wet cake was dried under moist N₂. The final dried Ca salt title product was isolated in 96% yield.

### EXAMPLE 13

### Preparation of crystalline hydrated calcium salt of dihydroxy open acid simvastatin (Compound I) from dihydroxy open acid simvastatin ammonium salt

Method A: 50 g of dihydroxy open acid simvastatin ammonium salt was dissolved into 800 ml of 25% nPrOH which was then added dropwise to a solution of Ca(OAc)₂•H₂O (10.7 g) in 75 ml of water at room temperature over 2h. The resulting slurry was aged at 30 to 35°C h. After cooling to room temperature, the slurry was isolated by filtration. The wet cake was washed with 10% nPrOH (500 ml x 3). The wet cake was loaded with antioxidants and dried under moist N₂ as described above to give the title product.

Method B: 50 g of dihydroxy open acid simvastatin ammonium salt was added into a solution of Ca(OAc)₂•H₂O (10.7 g) in 1.5 L of 10% nPrOH in one portion at room temperature. The resulting slurry was aged at 30 to 35°C 5 h. After cooling to room temperature, the slurry was isolated by filtration. The wet cake was washed with 10% nPrOH (500 ml x 3). The wet cake was loaded with antioxidants and dried under moist N₂ as described above to give the title product.

By using both Methods A and B described as above, the title product could also prepared from ammonium salt in the following aqueous solvents: acetone, MeOH, EtOH, iPrOH, MeCN, neat water, DMF, DMSO, and similar solvents.

### EXAMPLE 14

### Recrystallization Procedure Using nPrOH-H₂O

Dried Compound I (21 g) was dissolved in 150ml of 40% nPrOH at 35°C and line filtered. This solution was added dropwise to a slurry of 10 wt% seed in 480 ml of 4% PrOH at 35 to 40°C over 3 to 5 h. After aging overnight at 35 to 40°C, the slurry was allowed to cool to room temperature. The solid was filtered and washed with 10% nPrOH (200ml x 2). The wet cake was dried under moist N₂. 95% yield.

### EXAMPLE 15

### Recrystallization Process Using EtOH-H₂O

Method A: 25 g of Compound I was dissolved into 425ml of 95% EtOH at 40°C and line filtered. The filtered solution was added dropwise to 825ml of water in the presence of 10% wt seed at 30 to 35°C over 3 to 5 h. The slurry was aged overnight and cooled to 0 to 5°C before filtration. The wet cake was washed with 250 ml of 30% EtOH and dried under moist N₂ at room temperature. 92% yield.

Method B: 25 g of Compound I was dissolved into 625ml of 95% EtOH at 30 to 40°C and line filtered. 525 ml of water was added at 30 to 40°C. After adding 10wt% seed, 825 ml of water was added dropwise at 30 to 40°C over 3 h. The slurry was aged overnight and cooled to 0 to 5°C before filtration. The wet cake was washed with 250 ml of 30% EtOH and dried under moist N₂ at room temperature. 92% yield.

### EXAMPLE 16

An open, randomized, four-period, crossover study to compare the effect of itraconazole on the single-dose pharmacokinetics of intraduodenally administered dihydroxy open acid simvastatin versus orally administered simvastatin in healthy male subjects.

Objectives: (1) To determine the effect of itraconazole, a potent CYP3A inhibitor, on the plasma AUC of active and of total HMG-CoA reductase inhibitory activity following a single intraduodenal dose of a solution containing 5 mg dihydroxy open acid simvastatin; (2) to determine and compare the dose-adjusted plasma AUC of active HMG-coA reductase inhibitory activity following a single intraduodenal dose of a solution containing 5 mg dihydroxy open acid simvastatin versus a single oral administration of simvastatin 20-mg film coated tablet (FCT); (3) to determine the effect of itraconazole on the plasma AUC's of dihydroxy open acid simvastatin and of simvastatin lactone concentration following a single intraduodenal dose of a solution containing 5 mg dihydroxy open acid simvastatin.

Study Design: This study was designed in open, four-period crossover randomized fashion. Twelve healthy male subjects received four treatments (A, B, C and D). In Treatment A, subjects received itraconazole 200 mg (2 x 100-mg capsule) for 4 days followed by a single dose of 5-mg dihydroxy open acid simvastatin solution administered intraduodenally on day 4, 1hour after the fourth daily dose of intraconazole. In Treatment B, subjects were given a single dose of 5-mg dihydroxy open acid simvastatin solution administered intraduodenally on day 1. Intraduodenal administration was accomplished via a nasoduodenal tube placed under flouroscopic guidance by an experienced gastroenterologist just prior to dosing and removed following the 1-hr postdose measurements. Treatments C and D were similar to those of Treatments A and B, except that orally dosed simvastatin 20-mg conventional film coated tablet was used. The wash out between treatment periods was at least 7-days following a treatment containing itraconazole or at least 3 days following a treatment without itraconazole. Plasma samples were collected at appropriate time intervals for to 24 hours following simvastatin or dihydroxy open acid simvastatin-administration, for analysis of total and active HMG-CoA reductase inhibitory activities as well as for simvastatin and dihydroxy open acid simvastatin concentrations.

Analytical Methodology: Plasma concentrations of simvastatin and dihydroxy open acid were determined simultaneously by an improved liquid chromatography/tandem mass spectrometry (LC/MS/MS) method using lovastatin and dihydroxy open acid lovastatin acid as internal standards. An enzymatic assay method was used to determine plasma concentrations of active and total (active plus potentially active) HMG-CoA reductase inhibitory activity.

Pharmacokinetics: The area under the plasma concentration-time profile from time zero to the last sampling time (AUC0-last) was calculated using linear trapezoidal rule. The apparent elimination rate constant (k) of simvastatin and dihydroxy open acid simvastatin was estimated by least-squares regression analysis of the log-linear portion of the simvastatin and dihydroxy open acid simvastatin concentration-time data, and the apparent elimination half-life (t_{1/2}) was calculated as t_{1/2} = 0.693/k. All calculations were based on designated sampling times or actual sampling times when they differed from the designated times by more than 10 minutes.

Discussion of Results: This was an open, randomized, four-period crossover study in twelve healthy male subjects. The results showed that intraduodenal administration of dihydroxy open acid simvastatin 5-mg solution yielded higher (∼4-fold) dose -adjusted plasma AUC of the active HMG-CoA reductase inhibitory activity than oral administration of simvastatin 20-mg tablet (Table 1). Following dihydroxy open acid simvastatin administration, the unchanged dihydroxy open acid simvastatin was the major component (∼60%), while simvastatin was a minor component (<10%) contributing to plasma HMG-CoA reductase inhibitory activity. As evident by comparable AUC values for both-the total and active inhibitors (see Table 1) as well as low plasma levels of simvastatin in plasma (AUC<10% of dihydroxy open acid simvastatin AUC) (see Table 3), lactonization of either dihydroxy open acid simvastatin or its active metabolites occurred minimally following intraduodenal administration of dihydroxy open acid simvastatin. Pre-treatment with itraconazole caused minimal changes (1.3-1.5-fold) in the systemic exposure as measured by AUC and Cₘₐₓ of HMG-CoA reductase inhibitory activity (total or active) following administration of dihydroxy open acid simvastatin 5-mg intraduodenally, as compared to that observed following oral administration of simvastatin 20-mg tablet (1.3-3.8-fold) (see Table 2). When measured s the unchanged drug, the effect of itraconazole observed following dihydroxy open acid simvastatin administration was also minimal (1.5-fold) and was much less than the corresponding measure obtained following simvastatin administration (19-fold) (see Table 3). A moderate effect (3.4 fold increase) was noted for the AUC and Cₘₐₓ of simvastatin following treatment with itraconazole prior to dihydroxy open acid simvastatin administration (see Table 3). However, apparent t_{1/2} values for dihydroxy open acid simvastatin or simvastatin were essentially unchanged by itraconazole (see Table 3). Overall, these results indicate that the pharmacokinetics of dihydroxy open acid simvastatin is less prone to alteration by itraconazole, a potent CYP3A inhibitor, than that of simvastatin in humans. From these results it appears that dihydroxy open acid simvastatin, although a substrate for CYP3A, is metabolized with a much lower intrinsic clearance than that of simvastatin in human liver microsomes.

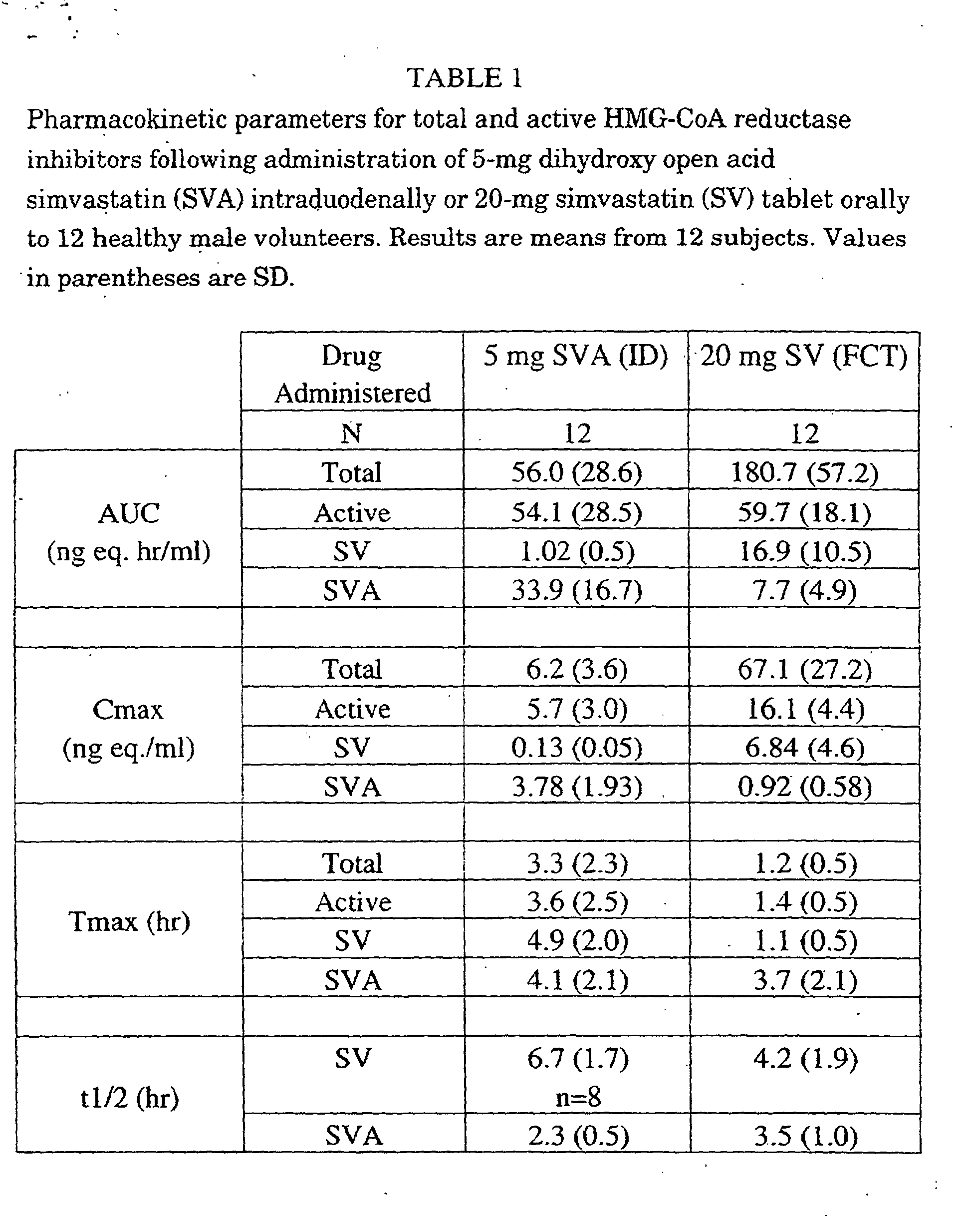

**TABLE 2**

| Pharmacokinetic parameters for total HMG-CoA reductase inhibitor following administration of 5-mg dihydroxy open acid simvastatin (SVA) intraduodenally or 20-mg simvastatin (SV) tablet orally with or without pretreatment with itraconazole (2x100-mg capsule, qd) for 4 days to 12 healthy male volunteers. Results are means from 12 subjects. Values in parentheses are SD. | | | |
|---|---|---|---|
| **Treatment** | **AUC** (ng eq.hr/mL) | **Cmax** (ng eq/mL) | **Tmax** (hr) |
| | | | |

| **Total Inhibitors** | | | |
|---|---|---|---|
| Single 5-mg SVA (ID) + 4 Days of Daily 200-mg Itraconazole | 86.1 ± 47 | 8.2 ± 4.0 | 4.3 ± 2.2 |
| Single 5-mg SVA (ID) | 56.0 ± 28.6 | 6.2 ± 3.6 | 3.3 ± 2.3 |
| **Geometric Mean Ratio** | **1.5** | **1.3** | |
| Single 20-mg SV (CT) + 4 Days of Daily 200-mg Itraconazole | 683 ± 232 | 126 ± 54.8 | 1.9 ± 0.9 |
| Single 20-mg SV (CT) | 180.7 ± 57.2 | 67.1 ± 27.2 | 1.2 ± 0.5 |
| **Geometric Mean Ratio** | **3.8** | **1.9** | |

| **Active Inhibitors** | | | |
|---|---|---|---|
| Single 5-mg SVA (ID) + 4 Days of Daily 200-mg Itraconazole | 75.0 ± 39.6 | 7.4 ± 3.9 | 5.5 ± 1.7 |
| Single 5-mg SVA (ID) | 54.1 ± 28.5 | 5.7 = 3.0 | 3.6 ± 2.5 |
| **Geometric Mean Ratio** | **1.3** | **1.3** | |
| Single 20-mg SV (CT) + 4 Days of Daily 200-mg Itraconazole | 195 ± 104 | 23.0 ± 13.6 | 3.1 ± 0.9 |
| Single 20-mg SV (CT) | 59.7 ± 18.1 | 16.1 ± 4.4 | 1.4 ± 0.5 |
| **Geometric Mean Ratio** | **3.1** | **1.3** | |

**TABLE 3**

| Pharmacokinetic parameters for simvastatin or dihydroxy open acid simvastatin following administration of 5-mg dihydroxy open acid simvastatin (SVA) intraduodenally or 20-mg simvastatin (SV) tablet orally with or without pretreatment with itraconazole (2x100-mg capsule, qd) for 4 days to 12 healthy male volunteers. Results are means from 12 subjects. Values in parentheses are SD. | | | | |
|---|---|---|---|---|
| **Treatment** | **AUC** (ng eq.hr/mL) | **Cmax** (ng cq/mL) | **t1/2** (hr) | **Tmax** (hr) |
| | | | | |

| **Simvastatin** | | | | |
|---|---|---|---|---|
| Single 5-mg SVA (ID) + 4 Days of Daily 200-mg Itraconazole | 5.18 ± 3.15 | 0.43 ± 0.2 | 6.7 ± 2.5 (n=11) | 5.3 ± 1.9 |
| Single 5₋mg SVA (ID) | 1.02 ± 0.5 | 0.13 ± 0.05 | 6.7 ± 1.7 (n=8) | 4.9 ± 2.0 |
| **Geometric Mean Ratio** | **4.4** | **3.2** | **1.0** | |
| Single 20-mg SV (CT) + 4 Days of Daily 200-mg Itraconazole | 316 ± 142 | 77.2 ± 50.7 | 5.2 ± 1.5 | 1.8 ± 1.1 |
| Single 20-mg SV (CT) | 16.9 ± 10.5 | 6.84 ± 4.6 | 4.2 ± 1.9 | 1.1 ± 0.5 |
| **Geometric Mean Ratio** | **19.4** | **11.4** | **1.3** | |

| **Simyastatin Acid** | | | | |
|---|---|---|---|---|
| Single 5-mg SVA (ID) + 4 Days of Daily 200-mg Itraconazole | 56.8 ± 33.5 | 5.78 ± 3.27 | 2.8 ± 1.1 | 4.7 ± 2.4 |
| Single 5-mg SVA (ID) | 33.9 ± 16.7 | 3.78 ± 1.93 | 2.3 ± 0.5 | 4.1 ± 2.1 |
| **Geometric Mean Ratio** | **1.5** | **1.5** | **1.1** | |
| Single 20-mg SV (CT) + 4 Days of Daily 200-mg Itraconazole | 86.2 ± 65.6 | 9.76 ± 7.38 | 4.5 ± 1.3 | 4.0 ± 1.4 |
| Single 20-mg SV (CT) | 7.70 ± 4.9 | 0.92 ± 0.58 | 3.5 ± 10 | 3.7 ± 2.1 |
| **Geometric Mean Ratio** | **11.0** | **10.7** | **1.3** | |

### EXAMPLE 17

### Preparation of Enteric Coated Tablet Containing Compound I

Per a 200 mg tablet weight tablet, 20 mg of Sureteric® is dissolved in 113.4 microliter of water. Depending on the batch size, appropriate level of film coating suspension is made and sprayed on tablets at ∼40°C using a pan coating process. Tablet weight gain is monitored throughout the operation to assure appropriate level of film coating on tablets.

## Claims

1. A crystalline hydrated form of the calcium salt of dihydroxy open acid simvastatin having an x-ray powder diffraction pattern obtained using CuKα radiation **characterized by** reflections at d-spacings of 30.7, 24.6, 15.9, 11.2, 8.58, 7.31, 6.74, 6.06, 5.35, 5.09, 4.93, 4.60, 3.93, 3.84, 3.67, 3.51 and 3.28Å.

2. The compound of claim 1 having a thermogravimetry curve **characterized by** a 6.3% weight loss from ambient room temperature to a stable weight loss plateau at about 175°C obtained under a nitrogen flow at a heating rate of 10°C/minute.

3. The compound of claim 1 or claim 2 having a differential scanning calorimetry curve **characterized by** lower temperature endotherms with peak temperatures of 52, 77 and 100°C and associated heats of 48, 90 and 21 J/g, respectively, and higher temperature endotherms due to decomposition with peak temperatures of 222 and 241°C and associated heats of 163 and 92 J/g obtained under a nitrogen flow bubbled through 16.0°C water at a heating rate of 10°C/minute in an open cup.

4. A crystalline hydrated form of the calcium salt of dihydroxy open acid simvastatin having the solid-state ¹³C nuclear magnetic resonance spectrum shown in Figure 4 obtained using an NMR system operating at 100.6 MHz for ¹³C and 400.1 MHz for ¹H using the CP/MAS technique with variable-amplitude cross-polarization and total sideband suppression.

5. A pharmaceutical composition comprising a therapeutically effective amount of the compound of any one of the preceding claims and a pharmaceutically acceptable carrier.

6. The pharmaceutical composition of claim 5 additionally comprising BHA, propyl gallate or a mixture thereof.

7. The pharmaceutical composition of claim 5 or claim 6 adapted for oral administration.

8. The pharmaceutical composition of claim 7 formulated in a time controlled-release dosage form.

9. The pharmaceutical composition of claim 7 formulated in a drug delivery device comprised of:
(A) a compressed core prepared from an admixture comprising:
(i) a therapeutically effective amount of the compound of any one of claims 1 to 4; and
(ii) a polymer which upon hydration forms gelatinous microscopic particles; and
(B) a water insoluble, water impermeable polymeric coating comprising a polymer and a plasticizer, which surrounds and adheres to the core, the coating having a plurality of formed apertures exposing between 1 and 75% of the core surface;
and wherein the release rate of the compound from the device is a function of the number and size of the apertures.

10. The use of the compound of any one of claims 1 to 4 for the preparation of a medicament useful for inhibiting HMG-CoA reductase.

11. The use of the compound of any one of claims 1 to 4 for the preparation of a medicament useful for treating hypercholesterolemia.

12. The use of the compound of any one of claims 1 to 4 for the preparation of a medicament useful for treatment of artherosclerotic disease, or for preventing or reducing the risk of developing atherosclerotic disease in a person at risk for developing atherosclerotic disease.

13. The use as claimed in claim 12 wherein the atherosclerotic disease is selected from coronary heart disease, cardiovascular disease, cerebrovascular disease and peripheral vessel disease.

14. The use of the compound of any one of claims 1 to 4 for the preparation of a medicament useful for preventing or reducing the risk of occurrence or recurrence of an atherosclerotic disease event in a patient at risk for having an atherosclerotic disease event.

15. The use as claimed in claim 14 wherein the medicament is useful for preventing or reducing the risk of occurrence or recurrence of an atherosclerotic disease event in a patient who has atherosclerotic disease.

16. The use as claimed in claim 14 wherein the medicament is useful for preventing or reducing the risk of occurrence or recurrence of an atherosclerotic disease event in a patient at risk for developing atherosclerotic disease.

17. The use as claimed in any one of claims 14 to 17 wherein the atherosclerotic disease event is selected from a coronary heart disease event, a cerebrovascular event and intermittent claudication.

18. The use as claimed in claim 17 wherein the coronary heart disease event is selected from coronary heart disease death, myocardial infarction, and coronary revascularization procedures.

19. The use as claimed in claim 17 wherein the cerebrovascular event is selected from a cerebrovascular accident and a transient ischemic attack.

20. A process for making the compound of any one of claims 1 to 4 comprising the steps of:
A) combining a mixture of a salt of dihydroxy open acid simvastatin in an aqueous solvent with calcium acetate hydrate to form an amorphous calcium salt of dihydroxy open acid simvastatin, wherein the aqueous solvent is selected from water, an aqueous-protic organic solvent mixture and an aqueous-aprotic organic solvent mixture;
B) aging the resulting mixture at a temperature up to 50°C until turnover of the amorphous calcium salt of dihydroxy open acid simvastatin to the crystalline calcium salt of dihydroxy open acid simvastatin is complete;
C) recovering the crystalline calcium salt of dihydroxy open acid simvastatin; and
D) drying the recovered crystals under a moist atmosphere.

21. The process of claim 20 wherein all the steps are performed under an inert atmosphere.

22. The process of claim 20 or claim 21 wherein the aqueous solvent is an aqueous-protic organic solvent mixture.

23. The process of claim 22 wherein the protic organic solvent is ethanol, methanol, isopropyl alcohol or n-propyl alcohol.

24. The process of any one of claims 20 to 23 wherein the aqueous solvent is an aqueous n-propyl alcohol mixture.

25. The process of any one of claims 20 to 24 wherein in step (A), the salt of dihydroxy open acid simvastatin is a metal salt, and the pH of the mixture of the salt of dihydroxy open acid simvastatin in an aqueous solvent is adjusted to 6 to 11 prior to combining it with the calcium acetate hydrate.

26. The process of claim 25 wherein the pH is adjusted to 6 to 9.

27. The process of claim 26 wherein the pH is adjusted to 7 to 8.5.

28. The process of any one of claims 25 to 27 wherein the pH is adjusted by addition to the mixture of an acid selected from a mineral acid and acetic acid.

29. The process of any one of claims 20 to 24 wherein in step (A), the salt of dihydroxy open acid simvastatin is the ammonium salt.

30. The process of any one of claims 20 to 29 wherein in step (B), the mixture is aged at a temperature from 10°C to 50°C.

31. The process of claim 30 wherein in step (B), the mixture is aged at a temperature from room temperature to 50°C.

32. The process of claim 31 wherein in step (B), the mixture is aged at a temperature from 30°C to 40°C.

33. The process of any one of claims 20 to 32 wherein in step (D), the recovered crystals are dried under an inert moist atmosphere having a relative humidity of 30 to 70%.

34. The process of any one of claims 20 to 32 wherein the recovered crystals are dried under an inert moist atmosphere at a temperature in the range from 10 to 40°C.

35. The process of claim 34 wherein the recovered crystals are dried at a temperature in the range from 25 to 35°C.

36. The process of any one of claims 20 to 35 wherein in step (D), the recovered crystals are suction dried.

37. The process of any one of claims 20 to 36 wherein in step (A), an anti-oxidant is combined with the salt of dihydroxy open acid simvastatin and the calcium acetate hydrate in the aqueous solvent.

38. The process of any one of claims 20 to 37 wherein after step (C) and prior to step (D); an anti-oxidant is combined with the recovered-crystalline calcium salt of dihydroxy open acid simvastatin in an aqueous solvent and the resulting material is recovered by filtration.

39. The process of claim 37 or claim 38 wherein the anti-oxidant is selected from BHA, propyl gallate and combinations thereof.

## Patentansprüche

1. Eine kristalline hydratisierte Form des Calciumsalzes der offenen Simvastatin-Dihydroxy-Säure, welche ein durch Verwendung von CuKα-Strahlung erhaltenes Röntgenpulverbeugungsmuster besitzt, das durch Reflektionen bei d-Werten von 30,7, 24,6, 15,9, 11,2, 8,58, 7,31, 6,74, 6,06, 5,35, 5,09, 4,93, 4,60, 3,93, 3,84, 3,67, 3,51 und 3,28Å gekennzeichnet ist.

2. Die Verbindung nach Anspruch 1, die eine Thermogravimetriekurve besitzt, welche durch einen 6,3%igen Gewichtsverlust von Umgebungs-Raumtemperatur bis zu einem stabilen Gewichtsverlustplateau bei etwa 175°C gekennzeichnet ist, aufgenommen unter einem Stickstoffstrom bei einer Heizrate von 10°C/Minute.

3. Die Verbindung nach Anspruch 1 oder Anspruch 2, die eine Differentialscanningkalorimetriekurve besitzt, welche **gekennzeichnet ist durch** Endothermen bei niedrigerer Temperatur mit Spitzentemperaturen von 52, 77 und 100°C und damit verbundenen Wärmen von 48, 90 bzw. 21 J/g und **durch** zersetzungsbedingte Endothermen bei höherer Temperatur mit Spitzentemperaturen von 222 und 241°C und damit verbundenen Wärmen von 163 und 92 J/g, aufgenommen unter einem Stickstoffstrom, der **durch** 16,0°C-Wasser geleitet wurde, bei einer Heizrate von 10°C/Minute in einem offenen Gefäß.

4. Eine kristalline hydratisierte Form des Calciumsalzes der offenen Simvastatin-Dihydroxy-Säure, welche das in Figur 4 gezeigte kernmagnetische Festphasen-¹³C-Resonanzspektrum besitzt, aufgenommen unter Verwendung eines NMR-Systems, das bei 100,6 MHz für ¹³C und bei 400,1 MHz für ¹H betrieben wird, wobei die CP/MAS-Technik mit Kreuzpolarisation bei variabler Amplitude und mit vollständiger Seitenbandunterdrückung verwendet wird.

5. Eine pharmazeutische Zusammensetzung, die eine therapeutisch wirksame Menge der Verbindung nach irgendeinem der vorhergehenden Ansprüche und einen pharmazeutisch annehmbaren Träger enthält.

6. Eine pharmazeutische Zusammensetzung nach Anspruch 5, die zusätzlich BHA, Propylgallat oder eine Mischung davon enthält.

7. Die pharmazeutische Zusammensetzung nach Anspruch 5 oder Anspruch 6, die zur oralen Verabreichung hergerichtet ist.

8. Die pharmazeutische Zusammensetzung nach Anspruch 7, die in einer Dosierungsform mit zeitgesteuerter Freisetzung formuliert ist.

9. Die pharmazeutische Zusammensetzung nach Anspruch 7, die in einer Arzneistofffreigabevorrichtung formuliert ist, umfassend:
(A) einen gepreßten Kern, hergestellt aus einer Mischung, die enthält:
(i) eine therapeutisch wirksame Menge der Verbindung nach irgendeinem der Ansprüche 1 bis 4 und
(ii) ein Polymer, das bei der Hydratisierung gelartige mikroskopische Teilchen bildet, und
(B) einen wasserunlöslichen, wasserundurchdringbaren polymeren Überzug, der ein Polymer und ein Plastifizierungsmittel enthält und der den Kern umgibt und daran haftet, wobei der Überzug eine Mehrzahl geformter Öffnungen enthält, die zwischen 1 und 75% der Kernoberfläche freilegen,
und wobei die Freigaberate der Verbindung von der Vorrichtung eine Funktion der Anzahl und Größe der Öffnungen ist.

10. Die Verwendung der Verbindung nach irgendeinem der Ansprüche 1 bis 4 zur Herstellung eines Medikaments, das sich zur Inhibierung von HMG-CoA-Reduktase eignet.

11. Die Verwendung der Verbindung nach irgendeinem der Ansprüche 1 bis 4 zur Herstellung eines Medikaments, das sich zur Behandlung von Hypercholesterinämie eignet.

12. Die Verwendung der Verbindung nach irgendeinem der Ansprüche 1 bis 4 zur Herstellung eines Medikaments, das sich zur Behandlung von artherosklerotischer Erkrankung oder zur Prävention oder Verringerung des Risikos der Ausbildung einer atherosklerotischen Erkrankung bei einer Person, die gefährdet ist, eine atherosklerotische Erkrankung auszubilden, eignet.

13. Die wie in Anspruch 12 beanspruchte Verwendung, wobei die atherosklerotische Erkrankung ausgewählt ist aus koronarer Herzerkrankung, kardiovaskulärer Erkrankung, zerebrovaskulärer Erkrankung und peripherer Gefäßerkrankung.

14. Die Verwendung der Verbindung nach irgendeinem der Ansprüche 1 bis 4 zur Herstellung eines Medikaments, das sich zur Prävention oder Verringerung des Risikos des Auftretens oder des erneuten Auftretens eines atherosklerotischen Erkrankungsfalles bei einem Patienten, der anfällig ist, einen atherosklerotischen Erkrankungsfall zu haben, eignet.

15. Die wie in Anspruch 14 beanspruchte Verwendung, wobei sich das Medikament zur Prävention oder Verringerung des Risikos des Auftretens oder des erneuten Auftretens eines atherosklerotischen Erkrankungsfalles bei einem Patienten, der an einer atherosklerotischen Erkrankung leidet, eignet.

16. Die wie in Anspruch 14 beanspruchte Verwendung, wobei sich das Medikament zur Prävention oder Verringerung des Risikos des Auftretens oder des erneuten Auftretens eines atherosklerotischen Erkrankungsfalles bei einem Patienten, der gefährdet ist, eine atherosklerotische Erkrankung auszubilden, eignet.

17. Die wie in irgendeinem der Ansprüche 14 bis 17 beanspruchte Verwendung, wobei der atherosklerotische Erkrankungsfall ausgewählt ist aus koronarem Herzerkrankungsfall, einem zerebrovaskulären Fall und intermittierendem Hinken.

18. Die wie in Anspruch 17 beanspruchte Verwendung, wobei der koronare Herzerkrankungsfall ausgewählt ist aus koronarem Herztod, Myokardinfarkt und Verfahren zur koronaren Revaskularisierung.

19. Die wie in Anspruch 17 beanspruchte Verwendung, wobei der zerebrovaskuläre Fall ausgewählt ist aus einem zerebrovaskulärem Unfall und einer transitorischen ischämischen Attacke.

20. Ein Verfahren zur Herstellung der Verbindung nach irgendeinem der Ansprüche 1 bis 4, umfassend die Schritte:
A) Vereinen einer Mischung aus einem Salz der offenen Simvastatin-Dihydroxy-Säure in einem wäßrigen Lösungsmittel mit Calciumacetat-Hydrat, um ein amorphes Calciumsalz der offenen Simvastatin-Dihydroxy-Säure zu ergeben, wobei das wäßrige Lösungsmittel ausgewählt ist aus Wasser, einem wäßrig-protischen organischen Lösungsmittelgemisch und einem wäßrigaprotischen organischen Lösungsmittelgemisch,
B) Altern der resultierenden Mischung bei einer Temperatur von bis zu 50°C, bis der Übergang des amorphen Calciumsalzes der offenen Simvastatin-Dihydroxy-Säure in das kristalline Calciumsalz der offenen Simvastatin-Dihydroxy-Säure vollständig ist,
C) Gewinnen des kristallinen Calciumsalzes der offenen Simvastatin-Dihydroxy-Säure und
D) Trocknen der gewonnenen Kristalle unter einer feuchten Atmosphäre.

21. Das Verfahren nach Anspruch 20, wobei alle Schritte unter einer inerten Atmosphäre durchgeführt werden.

22. Das Verfahren nach Anspruch 20 oder Anspruch 21, wobei das wäßrige Lösungsmittel ein wäßrig-protisches organisches Lösungsmittelgemisch ist.

23. Das Verfahren nach Anspruch 22, wobei das protische organische Lösungsmittel Ethanol, Methanol, Isopropylalkohol oder n-Propylalkohol ist.

24. Das Verfahren nach irgendeinem der Ansprüche 20 bis 23, wobei das wäßrige Lösungsmittel ein wäßriges n-Propylalkoholgemisch ist.

25. Das Verfahren nach irgendeinem der Ansprüche 20 bis 24, wobei in Schritt (A) das Salz der offenen Simvastatin-Dihydroxy-Säure ein Metallsalz ist und der pH-Wert der Mischung aus dem Salz der offenen Simvastatin-Dihydroxy-Säure in einem wäßrigen Lösungsmittel auf 6 bis 11 eingestellt wird, bevor sie mit dem Calciumacetat-Hydrat vereint wird.

26. Das Verfahren nach Anspruch 25, wobei der pH-Wert auf 6 bis 9 eingestellt wird.

27. Das Verfahren nach Anspruch 26, wobei der pH-Wert auf 7 bis 8,5 eingestellt wird.

28. Das Verfahren nach irgendeinem der Ansprüche 25 bis 27, wobei der pH-Wert durch Zugabe einer Säure, ausgewählt aus einer Mineralsäure und Essigsäure, zu der Mischung eingestellt wird.

29. Das Verfahren nach irgendeinem der Ansprüche 20 bis 24, wobei in Schritt (A) das Salz der offenen Simvastatin-Dihydroxy-Säure das Ammoniumsalz ist.

30. Das Verfahren nach irgendeinem der Ansprüche 20 bis 29, wobei in Schritt (B) die Mischung bei einer Temperatur von 10°C bis 50°C gealtert wird.

31. Das Verfahren nach Anspruch 30, wobei in Schritt (B) die Mischung bei einer Temperatur von Raumtemperatur bis 50°C gealtert wird.

32. Das Verfahren nach Anspruch 31, wobei in Schritt (B) die Mischung bei einer Temperatur von 30°C bis 40°C gealtert wird.

33. Das Verfahren nach irgendeinem der Ansprüche 20 bis 32, wobei in Schritt (D) die gewonnenen Kristalle unter einer inerten feuchten Atmosphäre mit einer relativen Feuchtigkeit von 30 bis 70% getrocknet werden.

34. Das Verfahren nach irgendeinem der Ansprüche 20 bis 32, wobei die gewonnenen Kristalle unter einer inerten feuchten Atmosphäre bei einer Temperatur im Bereich von 10 bis 40°C getrocknet werden.

35. Das Verfahren nach Anspruch 34, wobei die gewonnenen Kristalle bei einer Temperatur im Bereich von 25 bis 35°C getrocknet werden.

36. Das Verfahren nach irgendeinem der Ansprüche 20 bis 35, wobei in Schritt (D) die gewonnenen Kristalle sauggetrocknet werden.

37. Das Verfahren nach irgendeinem der Ansprüche 20 bis 36, wobei in Schritt (A) ein Antioxidationsmittel mit dem Salz der offenen Simvastatin-Dihydroxy-Säure und dem Calciumacetat-Hydrat in dem wäßrigen Lösungsmittel vereint wird.

38. Das Verfahren nach irgendeinem der Ansprüche 20 bis 37, wobei nach Schritt (C) und vor Schritt (D) ein Antioxidationsmittel mit dem gewonnenen kristallinen Calciumsalz der offenen Simvastatin-Dihydroxy-Säure in einem wäßrigen Lösungsmittel vereint und das resultierende Material durch Filtration gewonnen wird.

39. Das Verfahren nach Anspruch 37 oder Anspruch 38, wobei das Antioxidationsmittel ausgewählt ist aus BHA, Propylgallat und Kombinationen davon.

## Revendications

1. Forme hydratée cristalline du sel de calcium de la dihydroxy-simvastatine sous la forme acide ouverte ayant un spectre de diffraction X sur poudre obtenu au moyen du rayonnement Kα du Cu **caractérisé par** des réflexions à des distances réticulaires d de 30,7, 24,6, 15,9, 11,2, 8,58, 7,31, 6,74, 6,06, 5,35, 5,09, 4,93, 4,60, 3,93, 3,84, 3,67, 3,51 et 3,28Å.

2. Composé selon la revendication 1 ayant une courbe thermo-gravimétrique **caractérisée par** une perte de poids de 6,3% de la température ambiante à un plateau de perte de poids stable à environ 175°C, obtenue sous un flux d'azote à une vitesse de montée en température de 10°C/minute.

3. Composé selon la revendication 1 ou la revendication 2 ayant une courbe d'analyse calorimétrique à balayage différentiel **caractérisée par** des pics endothermiques à basse température de 52, 77 et 100°C et des quantités de chaleur associées de 48, 90 et 21 J/g respectivement, et des pics endothermiques à haute température du fait de la décomposition de 222 et 241°C et des quantités de chaleur associées de 163 et 92 J/g, obtenue sous un flux d'azote que l'on fait barboter à travers une eau à 16,0°C et à une vitesse de montée en température de 10°C/minute dans une coupelle ouverte.

4. Forme hydratée cristalline du sel de calcium de la dihydroxy-simvastatine sous la forme acide ouverte ayant un spectre de résonance magnétique nucléaire de ¹³C à l'état solide représenté sur la figure 4, obtenu au moyen d'un système de RMN fonctionnant à 100,6 MHz pour ¹³C et à 400,1 MHz pour ¹H en utilisant la technique de polarisation croisée/rotation à l'angle magique avec une polarisation croisée d'amplitude variable et une suppression totale des bandes latérales.

5. Composition pharmaceutique comprenant une quantité efficace sur le plan thérapeutique du composé selon l'une quelconque des revendications précédentes et un véhicule pharmaceutiquement acceptable.

6. Composition pharmaceutique selon la revendication 5 comprenant en outre du BHA, du gallate de propyle, ou un mélange des deux.

7. Composition pharmaceutique selon la revendication 5 ou la revendication 6 conçue pour l'administration orale.

8. Composition pharmaceutique selon la revendication 7 formulée sous une forme galénique à libération contrôlée.

9. Composition pharmaceutique selon la revendication 7 formulée dans un dispositif de relargage de médicament composé de :
(A) un noyau compressé préparé à partir d'un mélange comprenant:
(i) une quantité efficace sur le plan thérapeutique du composé selon l'une quelconque des revendications 1 à 4 ; et
(ii) un polymère qui, après hydratation, forme des particules microscopiques gélatineuses ; et
(B) un enrobage polymère insoluble dans l'eau, imperméable à l'eau, comprenant un polymère et un plastifiant, qui entoure le noyau et y adhère, l'enrobage possédant plusieurs ouvertures formées exposant entre 1 et 75% de la surface du noyau ;
et dans laquelle la vitesse de relargage du composé du dispositif est fonction du nombre et de la taille des ouvertures.

10. Utilisation du composé selon l'une quelconque des revendications 1 à 4 pour la préparation d'un médicament utile pour inhiber la HMG-CoA réductase.

11. Utilisation du composé selon l'une quelconque des revendications 1 à 4 pour la préparation d'un médicament utile pour traiter l'hypercholestérolémie.

12. Utilisation du composé selon l'une quelconque des revendications 1 à 4 pour la préparation d'un médicament utile pour traiter une maladie athéroscléreuse, ou pour prévenir ou réduire le risque de contracter une maladie athéroscléreuse chez une personne présentant un risque de contracter une maladie athéroscléreuse.

13. Utilisation selon la revendication 12 dans laquelle la maladie athéroscléreuse est choisie parmi une maladie coronarienne, une maladie cardio-vasculaire, une maladie vasculaire cérébrale et une maladie des vaisseaux périphériques.

14. Utilisation du composé selon l'une quelconque des revendications 1 à 4 pour la préparation d'un médicament utile pour prévenir ou réduire le risque d'apparition ou de récurrence d'une manifestation pathologique athéroscléreuse chez un patient présentant un risque de manifestation pathologique athéroscléreuse.

15. Utilisation selon la revendication 14 dans laquelle le médicament est utile pour prévenir ou réduire le risque d'apparition ou de récurrence d'une manifestation pathologique athéroscléreuse chez un patient souffrant d'une maladie athéroscléreuse.

16. Utilisation selon la revendication 14 dans laquelle le médicament est utile pour prévenir ou réduire le risque d'apparition ou de récurrence d'une manifestation pathologique athéroscléreuse chez un patient présentant un risque de contracter une maladie athéroscléreuse.

17. Utilisation selon l'une quelconque des revendications 14 à 17 dans laquelle la manifestation pathologique athéroscléreuse est choisie parmi une manifestation pathologique coronarienne et une claudication intermittente.

18. Utilisation selon la revendication 17 dans laquelle la manifestation pathologique coronarienne est choisie parmi la mort par maladie coronarienne, l'infarctus du myocarde et les techniques de revascularisation coronarienne.

19. Utilisation selon la revendication 17 dans laquelle la manifestation vasculaire cérébrale est choisie parmi un accident vasculaire cérébral et un accident ischémique transitoire.

20. Procédé de fabrication du composé selon l'une quelconque des revendications 1 à 4 comprenant les étapes consistant à :
A) associer un mélange d'un sel de la dihydroxy-simvastatine sous la forme acide ouverte dans un solvant aqueux avec de l'acétate de calcium hydraté pour former un sel de calcium amorphe de la dihydroxy-simvastatine sous la forme acide ouverte, dans lequel le solvant aqueux est choisi parmi l'eau, un mélange aqueux de solvant organique protique et un mélange aqueux de solvant organique aprotique ;
B) laisser vieillir le mélange ainsi obtenu à une température allant jusqu'à 50°C jusqu'à ce que la reconstitution du sel de calcium amorphe de la dihydroxy-simvastatine sous la forme acide ouverte en sel de calcium cristallin de la dihydroxy-simvastatine sous la forme acide ouverte soit terminée ;
C) récupérer le sel de calcium cristallin de la dihydroxy-simvastatine sous la forme acide ouverte ; et
D) sécher les cristaux récupérés sous une atmosphère humide.

21. Procédé selon la revendication 20 dans lequel toutes les étapes sont réalisées sous une atmosphère inerte.

22. Procédé selon la revendication 20 ou la revendication 21 dans lequel le solvant aqueux est un mélange aqueux de solvant organique protique.

23. Procédé selon la revendication 22 dans lequel le solvant organique protique est l'éthanol, le méthanol, l'alcool isopropylique ou l'alcool n-propylique.

24. Procédé selon l'une quelconque des revendications 20 à 23 dans lequel le solvant aqueux est un mélange aqueux d'alcool n-propylique.

25. Procédé selon l'une quelconque des revendications 20 à 24 dans lequel, dans l'étape (A), le sel d'acide ouvert dihydroxylé simvastatine est un sel métallique, et le pH du mélange du sel d'acide ouvert dihydroxylé simvastatine dans un solvant aqueux est ajusté à une valeur d 6 à 11 avant son association avec l'acétate de calcium hydraté.

26. Procédé selon la revendication 25 dans lequel le pH est ajusté à une valeur de 6 à 9.

27. Procédé selon la revendication 26 dans lequel le pH est ajusté à une valeur de 7 à 8,5.

28. Procédé selon l'une quelconque des revendications 25 à 27 dans lequel le pH est ajusté par addition au mélange d'un acide choisi parmi un acide minéral et l'acide acétique.

29. Procédé selon l'une quelconque des revendications 20 à 24 dans lequel, dans l'étape (A), le sel d'acide ouvert dihydroxylé simvastatine est le sel d'ammonium.

30. Procédé selon l'une quelconque des revendications 20 à 29 dans lequel, dans l'étape (B), le mélange est vieilli à une température de 10°C à 50°C.

31. Procédé selon la revendication 30 dans lequel, dans l'étape (B), le mélange est vieilli à une température allant de la température ambiante à 50°C.

32. Procédé selon la revendication 31 dans lequel, dans l'étape (B), le mélange est vieilli à une température allant de 30°C à 40°C.

33. Procédé selon l'une quelconque des revendications 20 à 32 dans lequel, dans l'étape (D), les cristaux récupérés sont séchés sous une atmosphère humide inerte ayant une humidité relative de 30 à 70%.

34. Procédé selon l'une quelconque des revendications 20 à 32 dans lequel les cristaux récupérés sont séchés sous une atmosphère humide inerte à une température dans la gamme de 10 à 40°C.

35. Procédé selon la revendication 34 dans lequel les cristaux récupérés sont séchés à une température dans la gamme de 25 à 35°C.

36. Procédé selon l'une quelconque des revendications 20 à 35 dans lequel, dans l'étape (D), les cristaux récupérés sont séchés sous aspiration.

37. Procédé selon l'une quelconque des revendications 20 à 36 dans lequel, dans l'étape (A), un antioxydant est associé au sel d'acide ouvert dihydroxylé simvastatine et à l'acétate de calcium hydraté dans le solvant aqueux.

38. Procédé selon l'une quelconque des revendications 20 à 37 dans lequel, après l'étape (C) et avant l'étape (D), un antioxydant est associé au sel de calcium cristallin d'acide ouvert dihydroxylé simvastatine récupéré, dans un solvant aqueux, et le produit ainsi obtenu est récupéré par filtration.

39. Procédé selon la revendication 37 ou la revendication 38 dans lequel l'antioxydant est choisi parmi le BHA, le gallate de propyle ou des combinaisons des deux.
